# EUROPEAN PATENT APPLICATION

(11) **EP 1 757 607 A1**
(43) Date of publication of application: **28.02.2007**
(21) Application number: 05018385.4
(22) Date of filing: 24.08.2005
(51) Int. Cl.: C07D 487/04, A61K 31/55, A61P 33/02

(54) **N5-substituted benzo¬2,3|azepino¬4,5-b|indol-6-ones for treating tropical diseases**

(71) Applicant: Molisa GmbH, 39106 Magdeburg (DE)
(72) Inventor: Stuhlmann, Friedrich, Dr., 39106 Magdeburg (DE); Jäger, Timo, Dr., 39106 Magdeburg (DE); Flohé, Leopold, Prof. Dr., 39106 Magdeburg (DE); Schinzer, Dieter, Prof. Dr., 39106 Magdeburg (DE)
(74) Representative: Boeters, Hans Dietrich

(57) **Abstract**

The present invention relates to novel N⁵-substituted-benzo[2,3]azepino[4,5-b]indol-6-ones of the general formula (1): 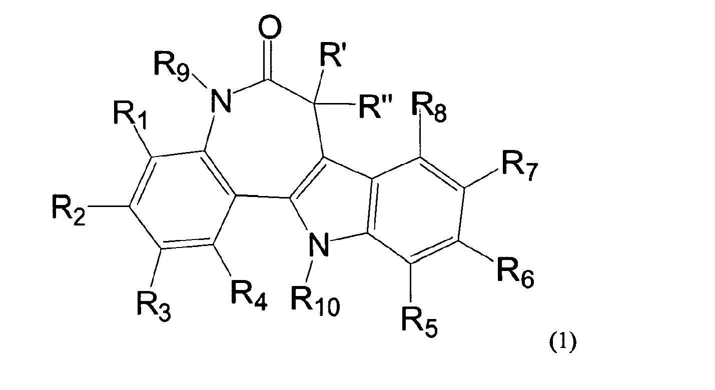 and pharmaceutically acceptable salts thereof, the use of these compounds as pharmaceutically active agents, especially for the prophylaxis and/or treatment of South American trypanosomiasis, African trypanosomiasis, sleeping sickness, Kala-Azar, visceral leishmaniasis, Baghdad boil or Aleppo boil, cutaneous leishmaniasis (CL), espundia, Chagas disease, mucocutaneous leishmaniasis (MCL), trichomoniasis, urogenital trichomonosis, giardiasis, lamblia dysentery, amoebiasis, primary amebic meningoencephalitis (PAM), keratitis or meningitis, coccidiosis, sarcosporidosis, toxoplasmosis, Malaria tropica, Malaria tertiana, Malaria quartana, pneumocystis carinii, pneumonia, pneumocystosis, Balantidium dysentery, and oriental sore. Furthermore, the present invention is directed towards pharmaceutical compositions containing at least one of the N⁵-substituted-benzo[2,3]azepino[4,5-b]indol-6-ones and/or pharmaceutically acceptable salts thereof.

## Description

The present invention relates to N⁵-substituted-benzo[2,3]azepino[4,5-b]indol-6-ones "paullones" and/or pharmaceutically acceptable salts thereof, the use of these compounds as pharmaceutically active agents, especially for prophylaxis and/or treatment of tropical diseases caused by *Balantidium coli, Eimeria* sp., *Entamoeba histolytica, Giardia intestinalis, Isospira* sp., *Leishmania* sp., *Naegleria fowleri, Plasmodium* sp., *Trichomonas vaginalis,* and *Trypanosoma* sp. such as Balantidium dysentery, coccidiosis, amoebiasis, giardiasis, visceral leishmaniasis (VL), cutaneous leishmaniasis (CL), mucocutaneous leishmaniasis (MCL), diffuse cutaneous leishmaniasis, primary amebic meningoencephalitis (PAM), malaria, trichomoniasis, African trypanosomiasis, and Chagas disease. Furthermore, the present invention is directed towards pharmaceutical compositions containing at least one of the N⁵-substituted-benzo[2,3]azepino[4,5-b]indol-6-ones and/or pharmaceutically acceptable salts thereof.

### Background of the invention

Paullones similar to the ones described in this patent are known from WO9965910, WO2004091663,20040186159,20030202959,20030181439,20030176484,20030125374.

### 2. Background

Trypanothione (TSH), the bis-glutathionyl derivative of spermidine, is a redox metabolite that is unique to kinetoplasts and some other protozoa. It was discovered in 1985 by Fairlamb and Cerami (1) and has since been discussed as a pivotal substitute for glutathione to sustain the redox balance in trypanosomatids (2).

A more recent breakthrough was the complete elucidation of the trypanothione-dependent peroxidase system in Crithidia fasciculata by Nogoceke et al. in 1997 (3). It is the most complex system to remove H₂O₂ and other hydroperoxides so far detected. It comprises a typical disulfide reductase (trypanothione reductase;TryR) for regeneration of reduced trypanothione from its cyclic disulfide form, tryparedoxin (TXN), which is remotely related to thioredoxin but requires trypanothione as reducing substrate, and finally peroxiredoxin-type peroxidases (TXNPx) that are reduced by TXN and acts on a large variety of hydroperoxides. Glutathione peroxidase-type proteins that are found in kinetoplasts are also reduced by TXN and thus ultimately depend on TSH too (for review see 4). The system is sustained by *de novo* synthesis of TSH from spermidine, glutathione and ATP, which, depending on species, involves one or two enzymes (glutathionyl-spermidine synthetase, GspS, and trypanothione synthetase,TryS). TryS is the only enzyme that catalyses the biosynthesis of TSH in *T.cruzi* (5) and *T.brucei* (6) and this is likely true also for *Leishmania* species (7). The TSH system has meanwhile been shown to similarly act in *T. cruzi, T. brucei* and *Leishmania* species (4). Three-dimensional structures of all proteins of the redox cascade have been obtained from several species (4,8), while the enzyme(s) synthetizing TSH still await structural elucidation.

Possibly even more important was the observation made by Dormeyer from Krauth-Siegel's group that one of the components of the peroxidase system, namely TXN, also serves as substrate of ribonucleotide reductase, the enzyme that generates the building blocks of DNA (9). This finding suggests that an inhibition of the trypanothione system should not only impair the parasites' resistance against oxidative stress but also their viability in general. All expectations based on the functional characterisation of the system were perfectly met when the individual enzymes were knocked out or knocked down by genetic techniques:
- A conditional knock out of trypanothione reductase in *T. brucei* resulted in enhanced peroxide sensitivity, growth arrest and loss of virulence in an animal infection model (10).
- Reduction of trypanothione reductase in *L. donovani* by a dominant negative approach led to impaired parasite survival in phagocytes (11).
- Knock-down of TXN and of the cytosolic form of TXNPx in *T. brucei* impaired viability (12).
- A knock-down of trypanothione synthetase in *T. brucei* led to a loss of trypanothione associated with gradual dying off of oxidatively unstressed parasites. At a stage of partial trypanothione depletion the parasites were already killed within 2 hours by a tiny flux of H₂O₂ mimicking the oxidative burst of phagocytes during the infection process (13) and these results have recently been confirmed by means of a different knock down system (7).
- In contrast, the mitochondrial form of TXNPx (14) and the glyoxalase II, which in kinetoplasts also works with TSH (15), proved not to be essential.

Although these target validations were, for technical reasons, predominantly performed in *T. brucei,* it can reasonably be assumed that the TSH system is equally important for *T. cruzi* and *Leishmania* species.

In short, most of the enzymes constituting the trypanosomal peroxidase system have been validated as drug targets and are structurally characterized for the design of specific inhibitors. TryS has equally been validated as drug target and appears particularly attractive, because its cellular abundance is low, its sequence does not display any significant similarity with known mammalian proteins and its inhibition would simultaneously abrogate all TSH-dependent processes in the pathogens, be they essential for survival or only supportive. Inhibition of TryS therefore appears to be the most efficient and safest approach to eradicate the parasites in African sleeping sickness, Chagas disease and the various forms of Leishmaniasis (6,7,13,16).

Paullones have been reported to inhibit various protein kinases and are therefore being discussed as potentially useful therapeutic agents, in particular for the treatment of malignant disease. The object of the present invention is to provide novel paullone that specifically inhibit the biosynthesis of trypanothione [N¹ ,N⁸ -bis(glutathionyl)spermidine], which is a compound that naturally occurs in pathogenic protozoa and is essential for their survival and/or virulence. Accordingly the present invention provides a powerful medicament against the infectious diseases caused by these microorganisms.

It is object of the present invention to provide compounds, stereoisomeric forms and/or pharmaceutically acceptable salts thereof which can be used as pharmaceutically active agents, especially for prophylaxis and/or treatment of tropical diseases caused by leishmania, trypanosome, protozoa, and plasmodium falciparum such as South American trypanosomiasis, African trypanosomiasis, sleeping sickness, Kala-Azar, visceral leishmaniasis, Baghdad boil or Aleppo boil, cutaneous leishmaniasis (CL), espundia, Chagas disease, mucocutaneous leishmaniasis (MCL), trichomoniasis, urogenital trichomonosis, giardiasis, lamblia dysentery, amoebiasis, primary amebic meningoencephalitis (PAM), keratitis or meningitis, coccidiosis, sarcosporidosis, toxoplasmosis, Malaria tropica, Malaria tertiana, Malaria quartana, pneumocystis carinii, pneumonia, pneumocystosis, Balantidium dysentery, and oriental sore.

The object of the present invention is solved by the teaching of the independent claims. Further advantageous features, aspects and details of the invention are evident from the dependent claims, the description, and the examples of the present application.

The novel N⁵-substituted-benzo[2,3]azepino[4,5-b]indol-6-ones "paullones" according to the present invention are represented by the following general formula (1) 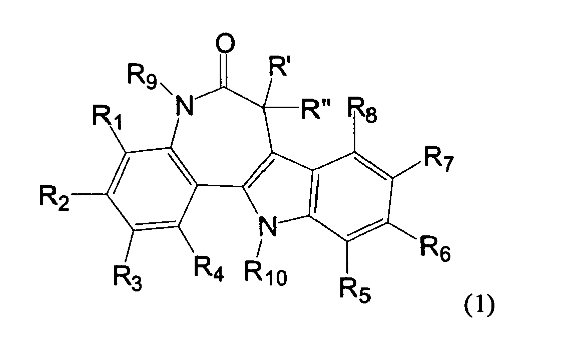 Wherein
R¹-R⁸, R⁸¹, R⁸² represent independently of each other -R¹¹, -R¹², -R¹³, -R¹⁴, -R¹⁵, -R¹⁶, -R¹⁷, -R¹⁸, -CH₂-R¹⁹, -CH₂-R²⁰, -CH₂-R²¹, -CH₂-R²², -CHR²³ R²⁴, -CHR²⁵R²⁶, -CHR²⁷R²⁸, -CHR²⁹ R³⁰, -CR³¹ R³² R³³, -CR³⁴ R³⁵ R³⁶, -CH₂-CH₂-R³⁷, -CH₂-CH₂-R³⁸, -CH₂-CH-R³⁹ R⁴⁰, -CH₂-CH-R⁴¹R⁴², -CR⁴³R⁴⁴-CR⁴⁵R⁴⁶R⁴⁷, -CR⁴⁸R⁴⁹-CR⁵⁰R⁵¹ R⁵², -(CH₂)ₛ-R⁶⁵, -CH₂)ₜ-R⁶⁶, -CR⁵³R⁵⁴-CR⁵⁵R⁵⁶-CR⁵⁷R⁵⁸R⁵⁹, -CR⁶⁰R⁶¹-CR⁶²R⁶³-CR⁶⁴R⁶⁵R⁶⁶, -(CH₂)_{q}-C R⁶⁰R⁶¹- (CH₂)ᵣR⁶⁴, -H;
R⁹ represents -CHR⁶⁸-CHR⁶⁷-CO-NR⁸¹R⁸², -CHR⁶⁷-CO-NR⁸¹R⁸², -CO-NR⁸¹R⁸², -CO-NR⁸¹R⁸², -CHR⁶⁸-CHR⁶⁷-CO-R⁸¹, -CHR⁶⁷-CO-R⁸¹, -CO-R⁸¹, -CHR⁶⁸-CHR⁶⁷-CO-OR⁸¹, -CHR⁶⁷-CO-OR⁸¹, -CO-OR⁸¹;
R' , R" and R¹⁰ represent independently of each other -H, -CPh₃, -CH₃, -C₂H₅, -C₃H₇, -cyclo-C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -C₇H₁₅, -C₈H₁₇, -C₉H₁₉, -C₁₀H₂₁, -Ph, -CH₂-Ph, -CH=CH₂, -CH₂-CH=CH₂, -C(CH₃)=CH₂, -CH=CH-CH₃, -C₂H₄-CH=CH₂, -CH=C(CH₃)₂, -C≡CH, -C≡C-CH₃, -CH₂-C≡CH;
R¹¹- R⁸⁰ represent independently of each other-H, -OH, -OCH₃, -OC₂H₅, -OC₃H₇ , -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -SC₃H₇ , -S-cyclo-C₃H₅, -SCH(CH₃)₂, -NO₂, -F, -Cl, -Br, -I, -N₃, -CN, -OCN, -NCO, -SCN, -NCS, -CHO, -COCH₃, -COC₂H₅, -COC₃H₇ , -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂ , -N(cyclo-C₃H₅)₂, -N[CH(CH₃)₂]₂, -N[C(CH₃)₃]₂, -SOCH₃, -SOC₂H₅, -SOC₃H₇ , -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃CH₃, -SO₃C₂H₅, -SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇ , -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇ , -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -O-CO-NH₂, -O-CONHCH₃, -O-CONHC₂H₅, -O-CONHC₃H₇, -O-CONH-cyclo-C₃H₅, -O-CONH[CH(CH₃)₂], -O-CONH[C(CH₃)₃], -O-CON(CH₃)₂, -O-CON(C₂H₅)₂, -O-CON(C₃H₇)₂, -O-CON(cyclo-C₃H₅)₂, -O-CON[CH(CH₃)₂]₂, -O-CON[C(CH₃)₃]₂, -CH₂F, -CF₂H, -CF₃, -CH₂Cl, -CHCl₂, -CCl₃, -CH₂Br, -CHBr₂, -CBr₃, -CPh₃, -CH₂-CH₂F, -CH₂-CHF₂, -CH₂-CF₃, -CH₂-CH₂Cl, -CH₂-CHCl₂, -CH₂-CCl₃, -CH₂-CH₂Br, -CH₂-CHBr₂, -CH₂-CBr₃, -CH₃, -C₂H₅, -C₃H₇ , -cyclo-C₃H₅, -CH(CH₃)₂, -C(CH₃)₃, -C₄H₉, -CH₂-CH(CH₃)₂, -CH(CH₃) -C₂H₅, -C₅H₁₁, -C₆H₁₃, -C₇H₁₅, -C₈H₁₇, -C₉H₁₉, -C₁₀H₂₁, -Ph, -CH₂-Ph, -CH=CH₂, -CH₂-CH=CH₂, -CH(CH₃)=CH₂), -CH=CH-CH₃, -C₂H₄-CH=CH₂, -CH=C(CH₃)₂, -C≡CH, -C≡C-CH₃, -CH₂-C≡CH_{;} -CH₂-CH₂S-CH₃, -CH₂-OH, -CH(OH)CH₃, -CH₂-NH₂, -CH₂-CO-NH₂, -CH₂-CH₂-CO-NH₂, -CH₂-SH, - (CH₂)₃-NH-C(=NH)NH₂, -(CH₂)₄-NH₂, -CH₂-COOH, -CH₂-CH₂-COOH;
R⁶⁷ and R⁶⁸ represent independently of each other -R⁶⁹ , -R⁷⁰, -CH₂-R⁶⁹, -CHR⁷⁰R⁷¹, -CR⁷²R⁷³R⁷⁴, -(CH₂)ₙ-R⁷⁵, -(CH₂)ₚ-CHR⁷⁶ R⁷⁷, -(CH₂)ₘCR⁷⁸ R⁷⁹R⁸⁰, -C₆H₄-R⁷², -CH₂-C₆H₄-R⁷³,
R⁶⁹ - R⁸⁰ also represent independently of each other 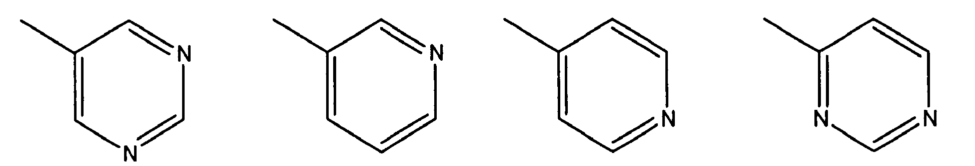 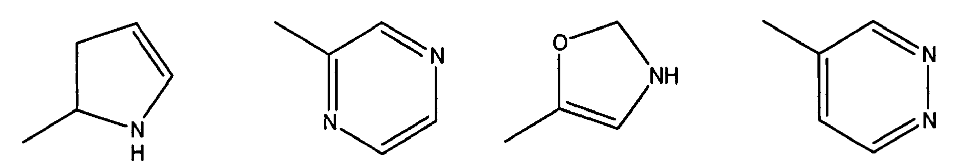 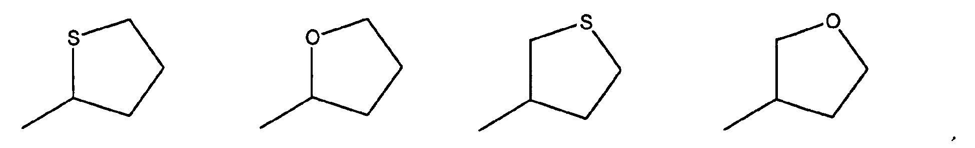 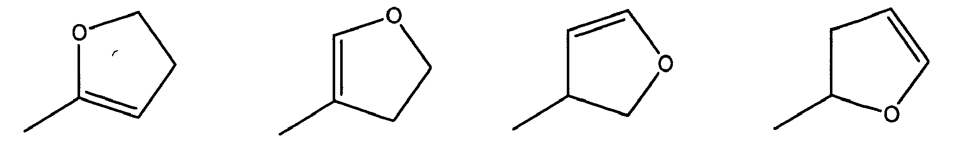 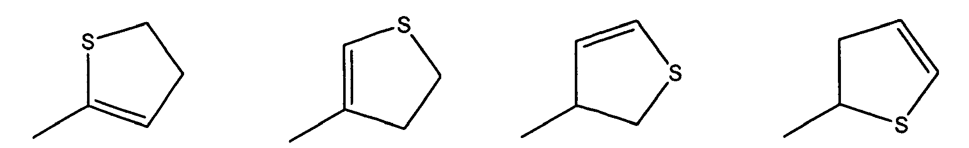 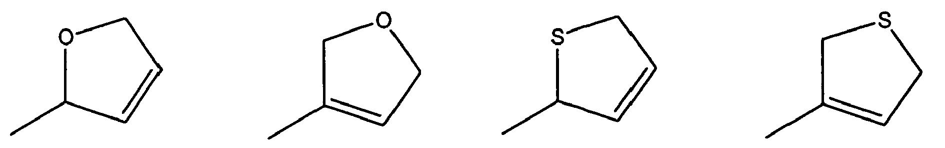 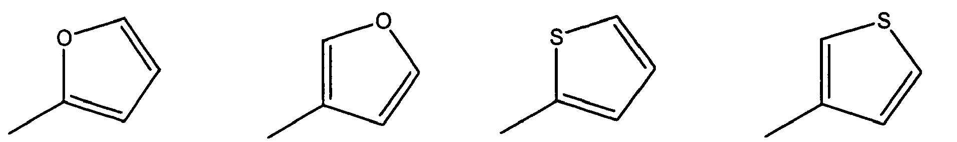 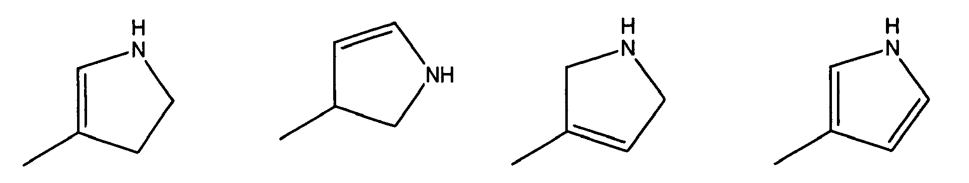 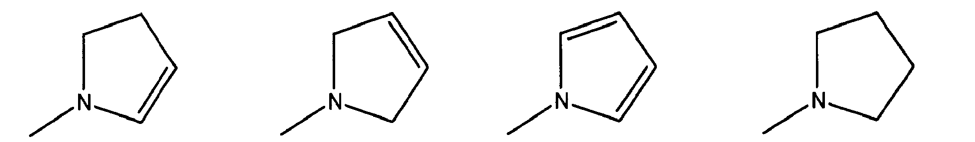 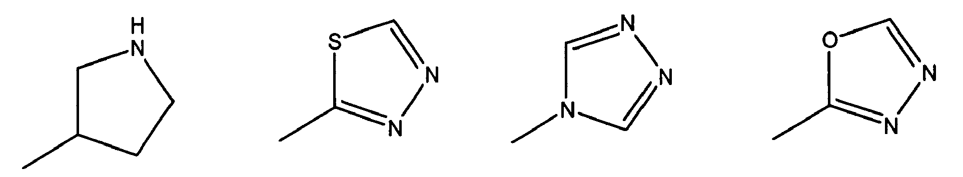 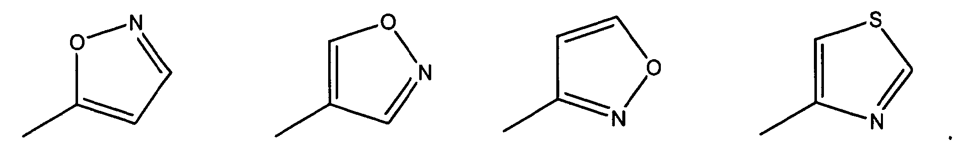 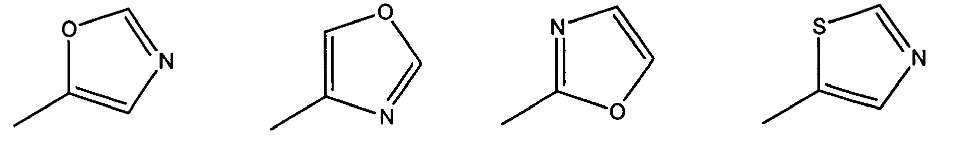 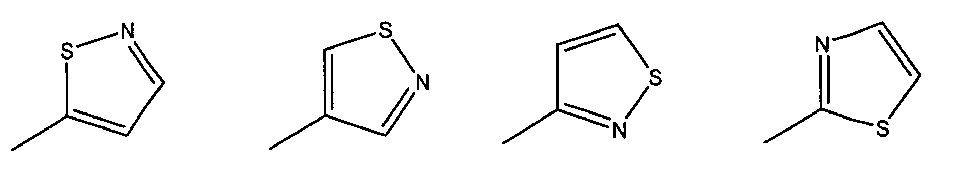 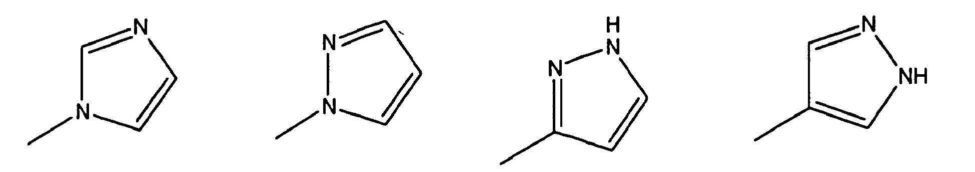 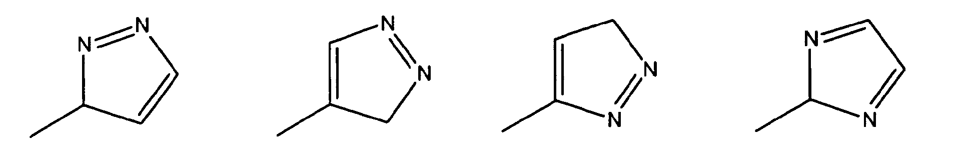 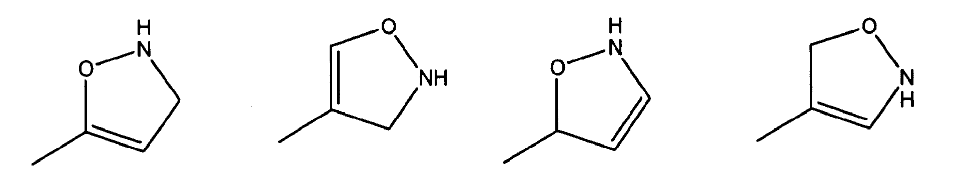 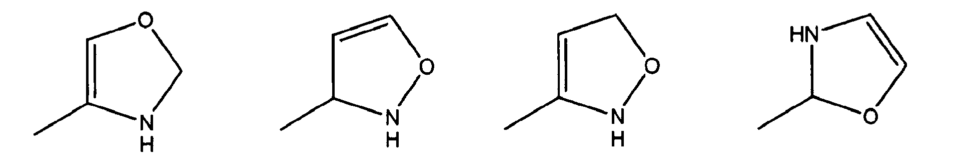 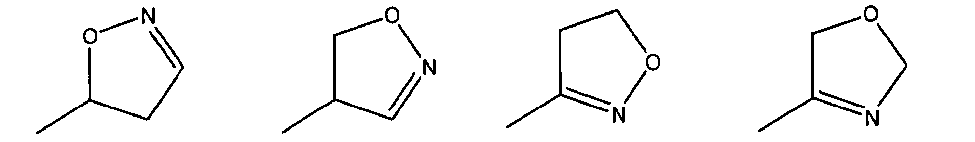 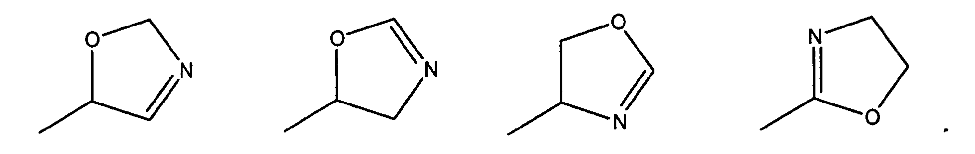 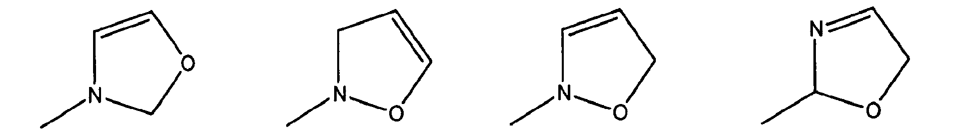 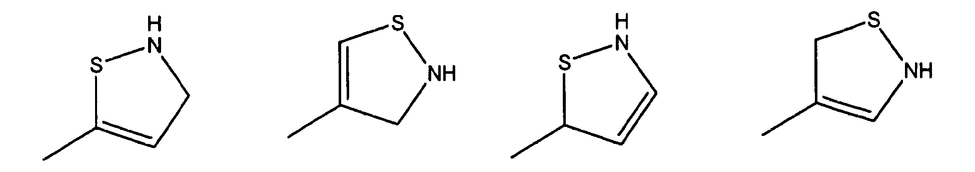 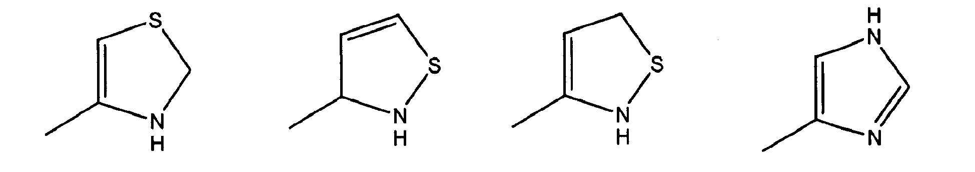 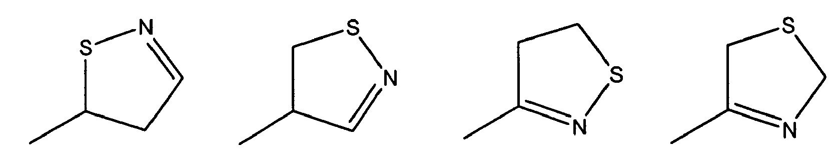 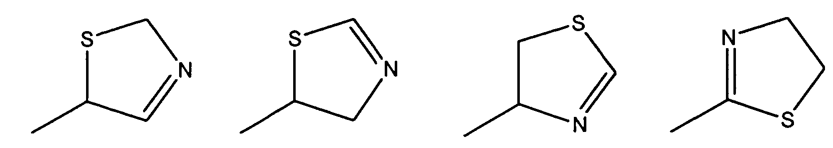 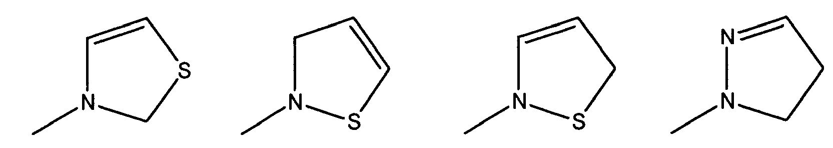 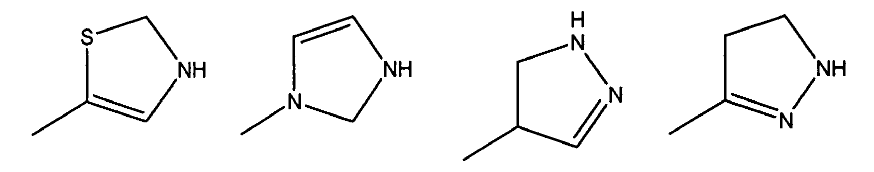 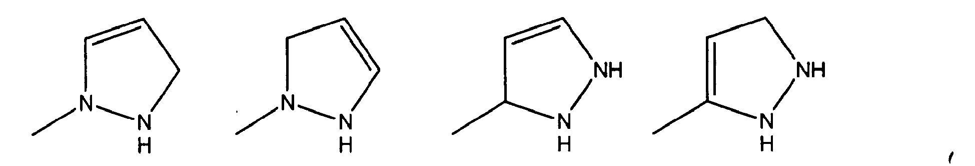 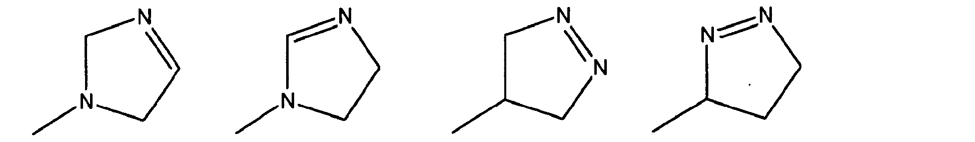 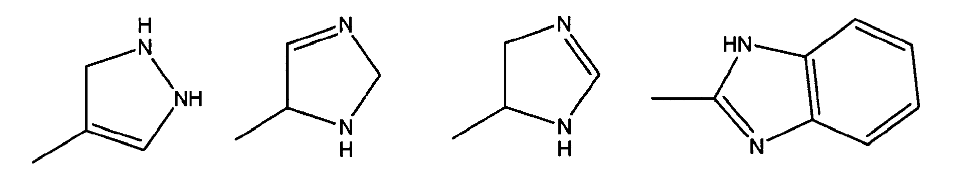 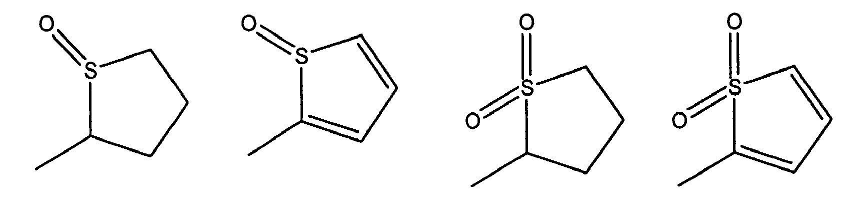 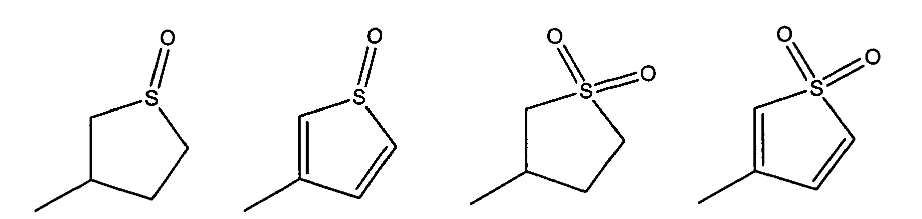 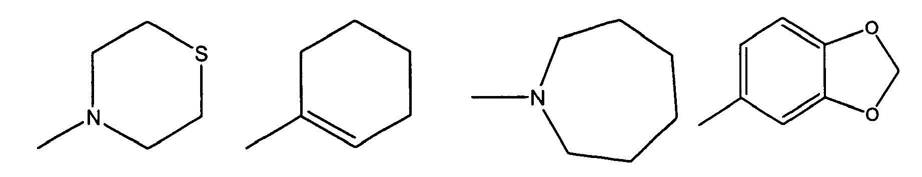 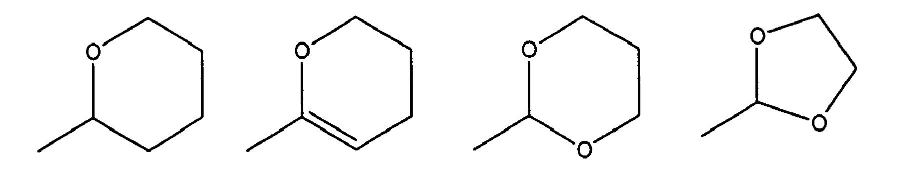 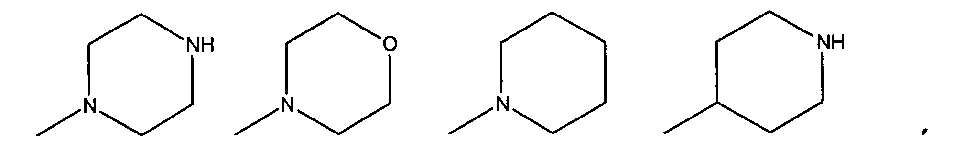 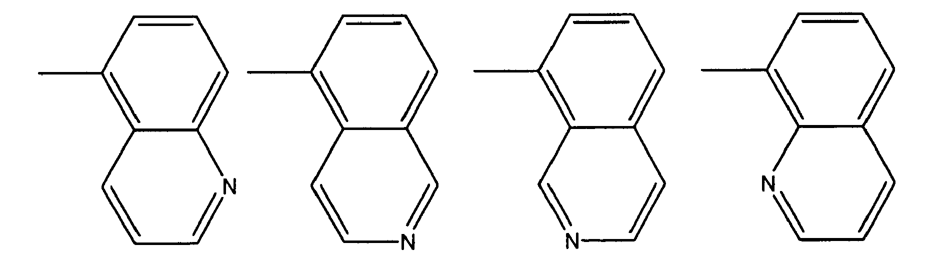 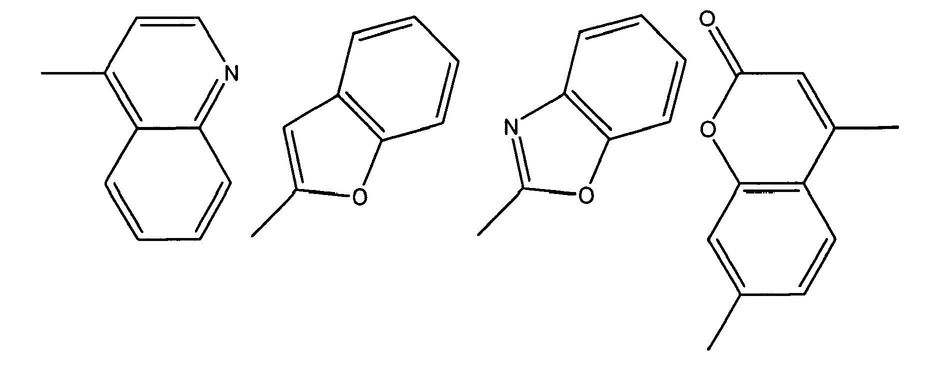 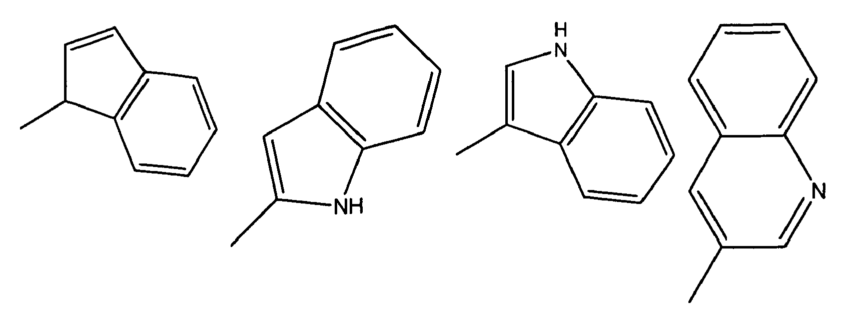 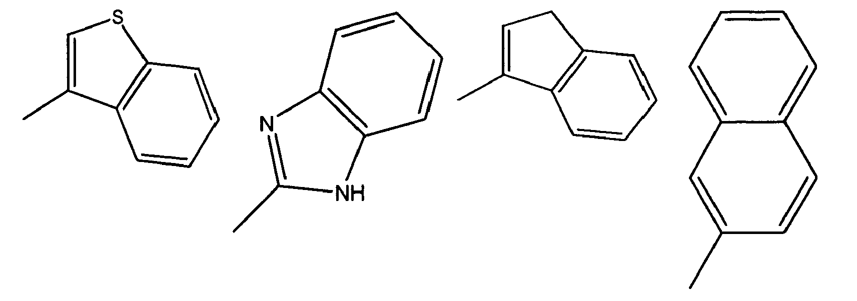 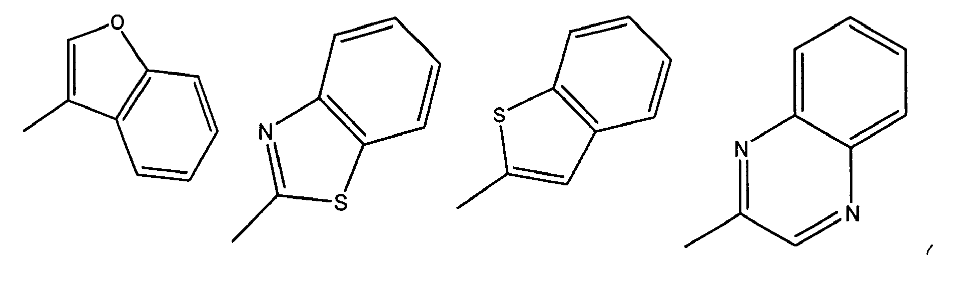 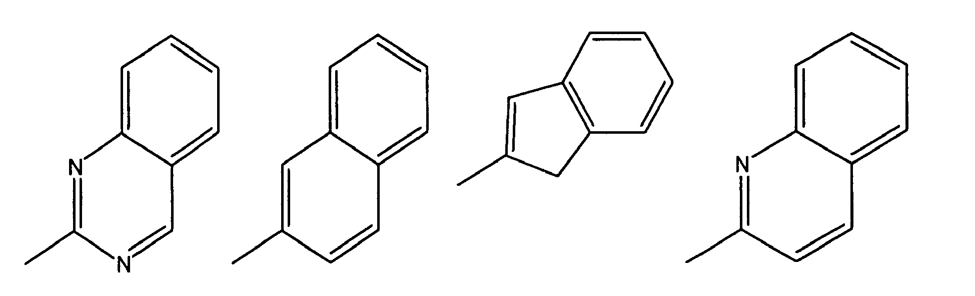 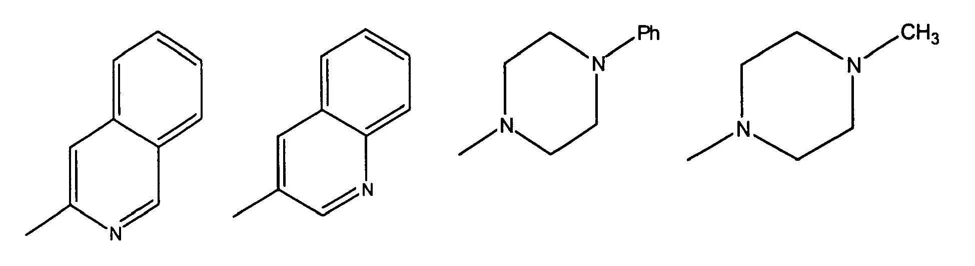
NR⁸¹R⁸² may also represent 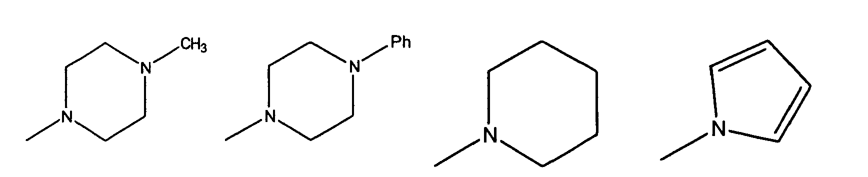 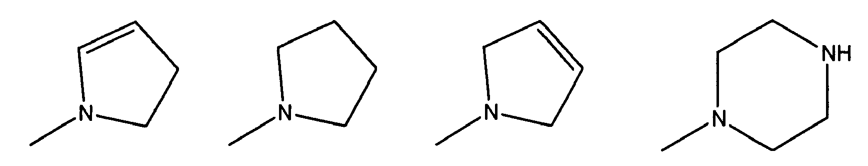 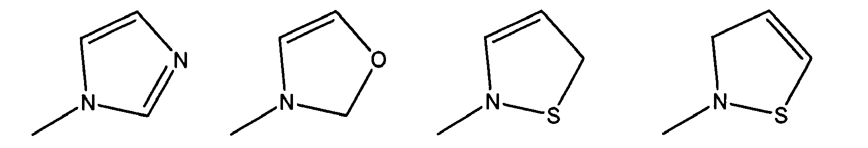 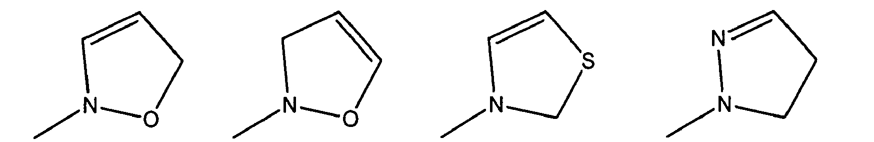 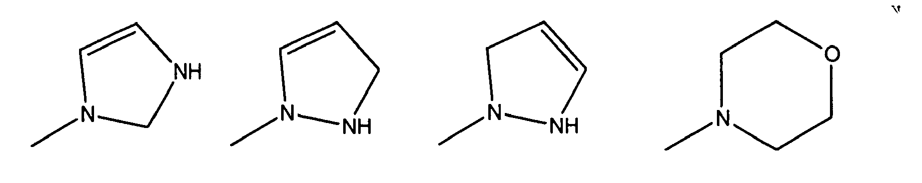 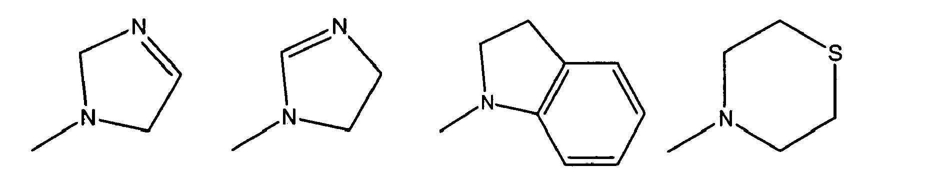
M, n, p, q, r, s, t are independently of each other integer from 0-10;
And stereoisomeric forms, prodrugs, solvates, hydrates and/or pharmaceutically acceptable salts thereof.

The following subformula (2) - 7) are especially preferred: 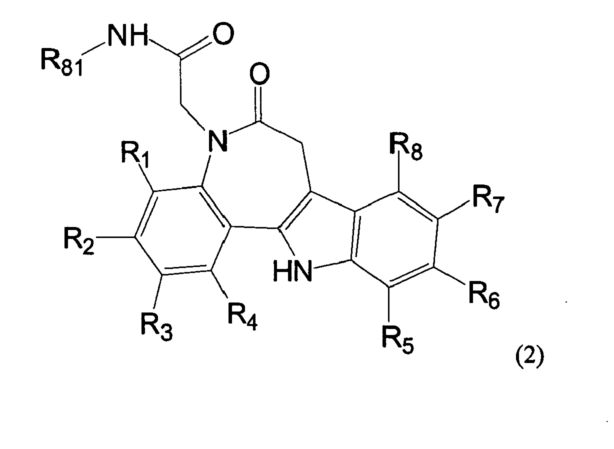 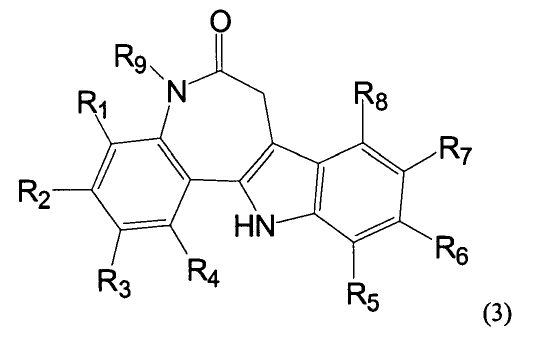 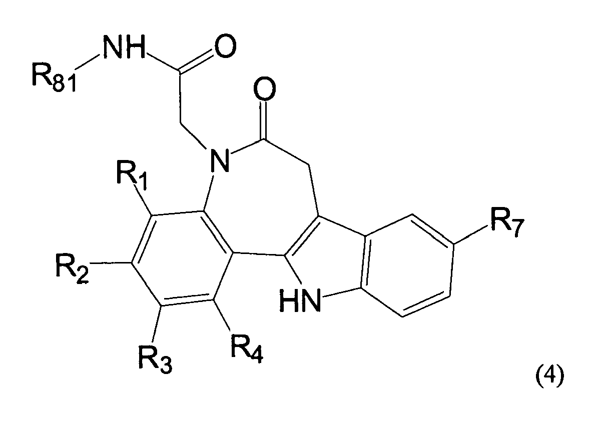 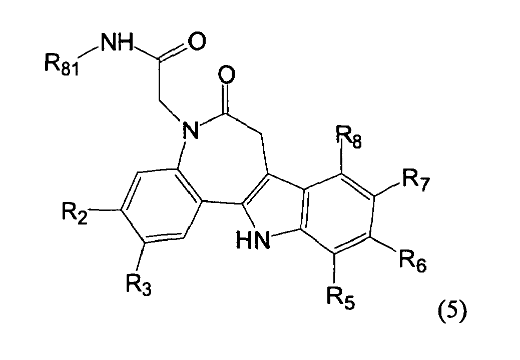 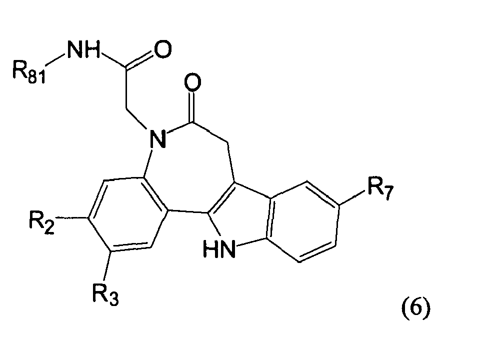 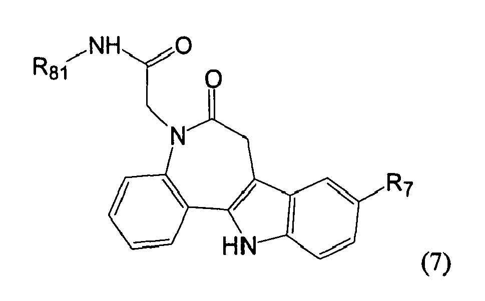 wherein R¹ - R⁹ and R¹¹ have the meaning as defined in claim 1.

In yet another preferred embodiment of the present invention the compound according to general formula (1) is selected from the group of compounds depicted in Table 1:

Table 1: Claimed compounds according to the present invention

| Comp. No. | IUPAC name | Formula | Inhibition of TS % |
|---|---|---|---|
| 13 | 2-(9-Bromo-6-oxo-6,7-dihydro-5*H*-benzo[2,3]azepino[4,5-b]indol-5-yl)-N-(2-methylamino-1-yl-ethyl)-acetamide | 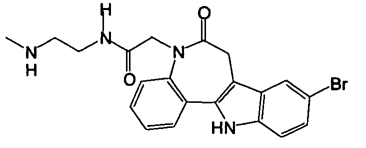 | 92.4 |
| 17 | N-(2-Amino-1-yl-ethyl)- 2-(9-bromo-6-oxo-6,7-dihydro-*5H*-benzo[2,3]azepino[4,5-b]indol-5-yl)-acetamide | 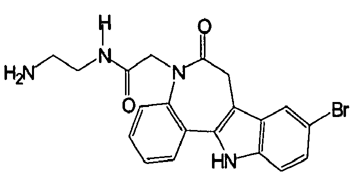 | 78.6 |
| 18 | N-(3-Amino-1-yl-propyl)-2-(9-bromo-6-oxo-6,7-dihydro-*5H*-benzo[2,3]azepino[4,5-b]indol-5-yl)-acetamide | 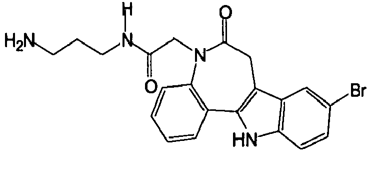 | 37.8 |
| 19 | N-(4-Amino-1-yl-butyl)-2-(9-bromo-6-oxo-6,7-dihydro-*5H*-benzo[2,3]azepino[4,5-b]indol-5-yl)-acetamide | 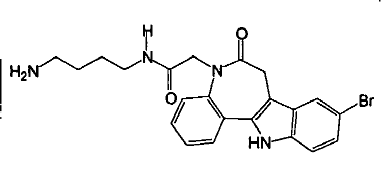 | 16.2 |
| 20 | N-(4-(3-Amino-1-yl-propylamino)-1-yl-butyl)-2-(9-bromo-6-oxo-6,7-dihydro-*5H*-benzo[2,3]azepino[4,5-b]indol-5-yl)-acetamide | 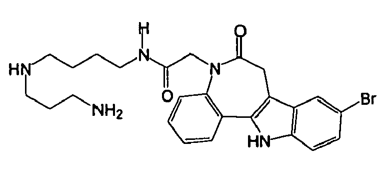 | 24.6 |
| 21 | N-(3-(4-Amino-1-yl-butylamino)-1-yl-propyl)-2-(9-bromo-6-oxo-6,7-dihydro-*5H*-benzo[2,3]azepino[4,5-b]indol-5-yl)-acetamide | 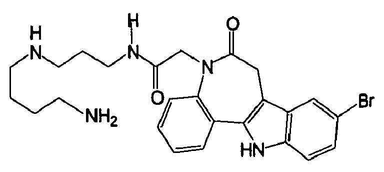 | 22.5 |
| 22 | 2-(9-Cyano-6-oxo-6,7-dihydro-5*H*-benzo[2,3]azepino[4,5-b]indol-5-yl)-N-(2-methylamino-1-yl-ethyl)-acetamide | 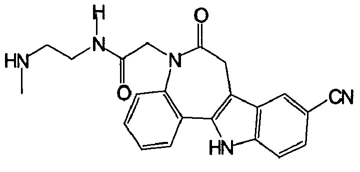 | 51.7 |
| 23 | N-(2-Methylamino-1-yl-ethyl)-2-(6-oxo-6,7-dihydro-5*H*-benzo[2,3]azepino[4,5-b]indol-5-yl)-acetamide | 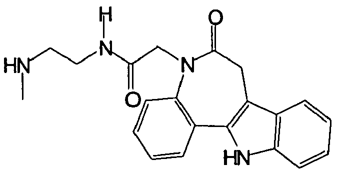 | 0 |
| 24 | 2-(9-Methyl-6-oxo-6,7-dihydro-5*H*-benzo[2,3]azepino[4,5-b]indol-5-yl)-N-(2-methylamino-1-yl-ethyl)-acetamide | 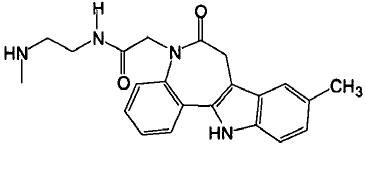 | 20.5 |
| 25 | N-[2-(9-Bromo-6-oxo-6,7-dihydro-5*H*-benzo[2,3]azepino[4,5-b]indol-5-yl)-acetyl]-piperazine | 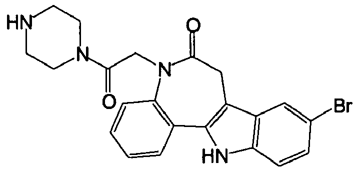 | 76.5 |
| 26 | 2-(9-Bromo-6-oxo-6,7-dihydro-5*H*-benzo[2,3]azepino[4,5-b]indol-5-yl)-acetic acid hydrazide | 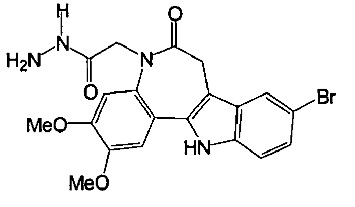 | 0 |

The results exhibited in Table 1 prove that the compounds of the present invention are potent pharmaceutically active agents against Trypanothione Synthetase TS.
The present invention also comprises pharmaceutically acceptable salts of the compounds according to the general formula (1), all stereoisomeric forms according to the general formula (1) as well as solvates, especially hydrates or prodrugs thereof. A prodrug is commonly described as an inactive or protected derivative of an active ingredient or a drug, which is converted to the active ingredient or drug in the body.

The compounds of the present invention are basic and may form salts with organic or inorganic acids. Examples of suitable acids for such acid addition salt formation are hydrochloric acid, hydrobromic acid, sulphuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner. In case, the compound bear acidic substituents, the formation of salts with inorganic or organic bases may be possible. Examples of such bases are NaOH, KOH, NH₄OH, tetraalkylammonium hydroxide, lysine or arginine and the like. Salts may be prepared in a conventional manner using methods well known in the art, for example by treatment of a solution of the compound of the general formula (1) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

In the case of chiral substituents, compounds of the general formula (1) may exist in the form of optical isomers, e.g. enantiomers, diastereomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (1) contains an alkene moiety, the alkene can be presented as a cis or trans isomer or mixture thereof. When an isomeric form of a compound of the invention is provided substantially free of other isomers, it will preferably contain less than 5% w/w, more preferably less than 2% w/w and especially less than 1% w/w of the other isomer(s). The afore-mentioned compounds are useful as pharmaceutically active agents, i.d. as drugs or medicine.
In a further aspect of the present invention, the novel compounds according to the general formula (1) are used as pharmaceutically active agent. Furthermore, the inventive benzo[2,3]azepino[4,5-b]indol-6-ones are identified as inhibitors of trypanothione synthetase, especially of trypanothione synthetases from Crithidia fasciculata, Trypanosoma brucei brucei, Trypanosoma cruzii, and Leishmania donovani.

The pharmaceutical compositions according to the present invention comprise at least one compound according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutaneous, rectal, subcutaneous, sublingual, topical, or transdermal application, which preparations in addition to typical vehicles and/or diluents contain at least one compound according to the present invention and/or a pharmaceutically acceptable salt thereof as active ingredient.

The pharmaceutical compositions according to the present invention containing at least one compound according to the present invention and/or pharmaceutically acceptable salts thereofas active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulphate, talc, mannitol, ethyl alcohol (liquid filled capsules) and the like. Moreover, suitable binders, lubricants, disintergrating agents and coloring agents may also be incorporated into the tablet or capsule. Powders and tablets may contain 5 to about 95-weight % of the paullone derivative according to the general formula (1) or analogues thereof or the respective pharmaceutically active salt as active ingredient.

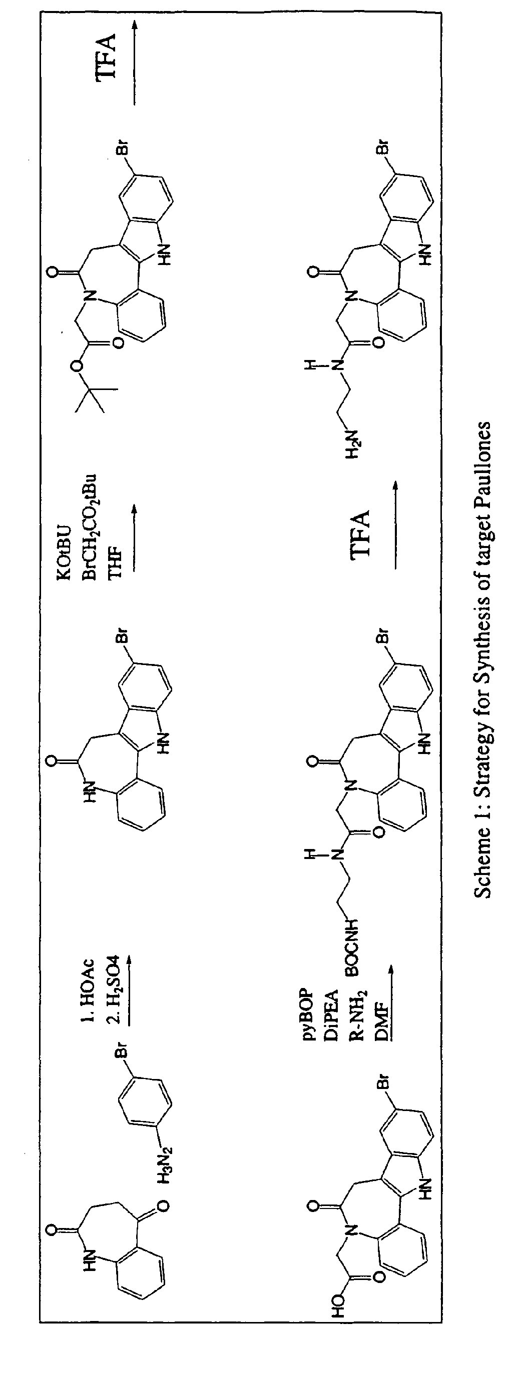

### EXPERIMENTAL PART:

### 2-(9-Bromo-6-oxo-6,7-dihydro-5H-benzo[2,3]azepino[4,5-b]indol-5-yl)-acetic acid tert.-butyl ester

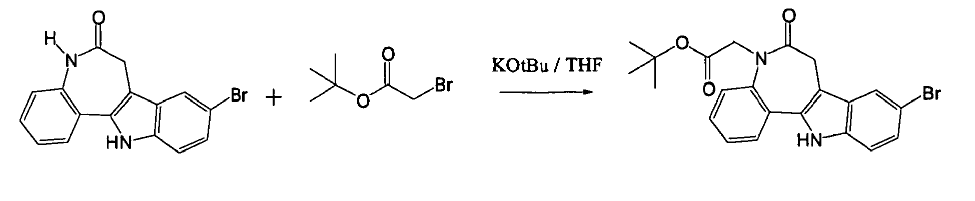

To 599 mg (1.8 mmole) of 9-Bromo-6-oxo-6,7-dihydro-5*H*-benzo[2,3]azepino[4,5-b]indole (MW 327) in 50 mL of dry THF are added 2 mL of a 1M solution of KOtBU in THF. Stirring is continued for 1 h. 2 mmoles (390 mg, 0.291 mL) of bromo acetic acid tert.-butyl ester are added. After stirring overnight, the THF is evaporated and the residue is distributed between 100 mL dichloromethane and 100 mL of water. After phase separation, drying of the organic phase over MgSO₄ and evaporation of the solvent, the residue is chromatographed on silica gel (27/1.5) using ether/pentane 1:1 as the elutant. The first fraction contains 58 mg (6%) of the compound with both nitrogen atoms alkylated, the second fraction is 295 mg (0.69 mmole; 39%; MW 425) of the title compound.
¹H-NMR (CDCl₃)
1.40 (s, 9H), 3.19 (br s, 1H), 3.90 (br s, 1H), 4.23 (br s, 1H), 4.44 (br s, 1H), 7.19-7.35 (m, 3H), 7.38-7.45 (m, 2H), 7.56 (d, 7.5 Hz, 1H), 7.72 (d, 1.1 Hz, 1H), 8.83 (s, 1H).
¹³C-NMR (CDCl₃)
171.2 q, 168.5 q, 139.9 q, 135.8 q, 133.1 q, 128.6 CH, 128.2 q, 126.4 CH, 125.7 CH, 125.55 CH, 125.4 q, 123.7 CH, 121.1 CH, 113.2 q, 112.6 CH, 110.24 q, 82.0 q, 53.6 CH₂, 31.7 CH₂, 27.8 CH.
X-ray analysis, HMBC and ¹H, ¹⁵N-HSQC-TOCSY confirmed the assigned structure in accordance with the corresponding methyl ester (Schultz et al., J. Med. Chem. 1999, 42, 2909-2919)

### 2-(9-Bromo-6-oxo-6,7-dihydro-5H-benzo[2,3]azepino[4,5-b]indol-5-yl)-acetic acid

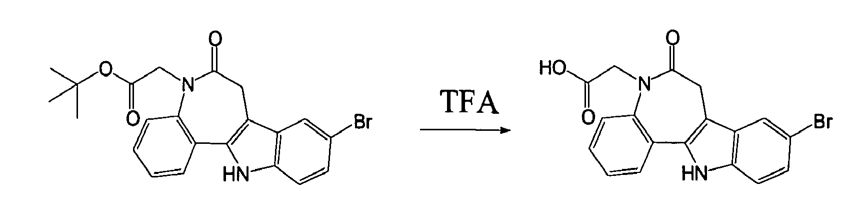

To 300 mg (0.68 mmoles; MW 425) of 2-(9-Bromo-6-oxo-6,7-dihydro-5*H-*benzo[2,3]azepino[4,5-b]indol-5-yl)-acetic acid tert.-butyl ester dissolved in dry dichloromethane are added 2 mL of freshly distilled trifluoroacetic acid. After stirring overnight the solvent is evaporated and the crude product is recrystallised from diethyl ether.
Yield 246 mg (0.64 mmoles; 95%; MW 386).
¹H-NMR (MeOH-d4)
3.11 (br s, 1H), 3.93 (br s, 1H), 4.20 (br s, 1H), 4.48 (br s, 1H), 7.20 (dd, 8.6 Hz, 1.8 Hz, 1H), 7.28-7.35 (m, 2H), 7.39 (td, 8.2 Hz, 1.3 Hz, 1H), 7.48 (dd, 8.2 Hz, 1.0 Hz, 1H), 7.65 (dd, 7.7 Hz, 1.5 Hz, 1H), 7.71 (d, 1.8 Hz, 1H).

### 2-(9-Bromo-6-oxo-6,7-dihydro-5H-benzo[2,3]azepino[4,5-b]indol-5-yl)-N-(2-tert.-butylaminocarbonyl-1-yl-ethyl)-acetamide

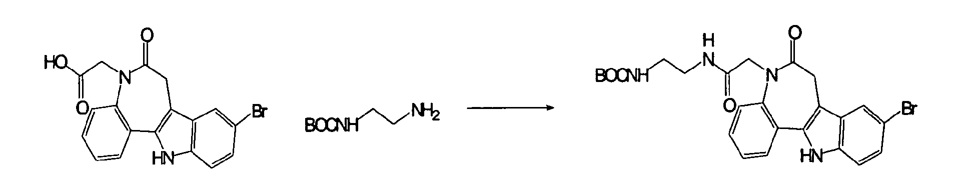

To 186 mg of 2-(9-Bromo-6-oxo-6,7-dihydro-*5H*-benzo[2,3]azepino[4,5-b]indol-5-yl)-acetic acid (0.48 mmoles; MW 386) in 1 mL of dry DMF cooled to 0°C are added 96 mg (0.6 mmoles) ofN-tert.-butyloxycarbonylethylene diamine, 283 mg (2.2 mmoles; 0.37 mL) of diisopropylethyl amine, and 286 mg ofpyBOP (0.55 mmoles). The resulting clear solution is stirred at room temperature for 24 h. Then the solution is distributed between 50 mL of 0.1 M HCL and ethyl acetate each. After separation of the phases the organic phase is washed with water, 0.1 M NaOH and water again. It is dried over MgSO₄ and evaporated to dryness.
Chromatography (silica gel; Toluene/Ether 2:1) and recrystallization from THF/toluene yields 121 mg (21%; 0.23 mmoles; MW 527) of the title compound.
¹H-NMR-Spectrum (MeOH-d4)
1.42 (s, 9H), 3.19 (br s, 3 H), 3.31 (br s, 2 H), 3.90 (br s, 1H), 4.07 (br s, 1H), 4.51 (br s, 1H), 7.28 (dd, 8.6 Hz, 1.8 Hz, 1H), 7.37-7.42 (m, 2H), 7.47 (td, 8.3 Hz, 1.3 Hz, 1H), 7.62 (d, 8.2 Hz, 1H), 7.73 (dd, 7.7 Hz, 1.6 Hz, 1H), 7.79 (d, 1.8 Hz, 1H).

### N-(2-Amino-1-yl-ethyl)-2-(9-bromo-6-oxo-6,7-dihydro-5H-benzo[2,3]azepino[4,5-b]indol-5-yl)-acetamide

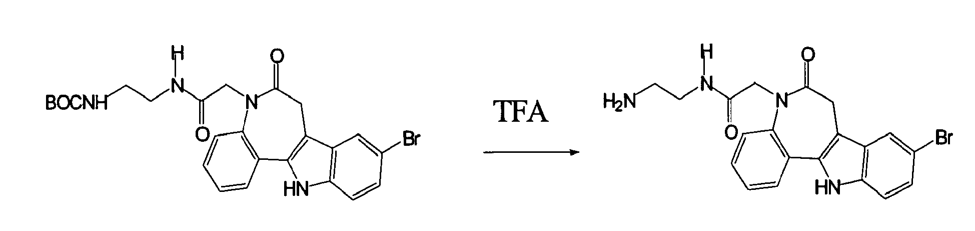

37 mg of 2-(9-Bromo-6-oxo-6,7-dihydro-5*H*-benzo[2,3]azepino[4,5-b]indol-5-yl)-N-(2-tert.-butylaminocarbonyl-1-yl-ethyl)-acetamide (0.070 mmoles; MW 527) are dissolved in 5 mL of dry dichloromethane. 1 mL of freshly distilled trifluoro acetic acid is added, and stirring is continued overnight. The solvent is evaporated and the oily residue dissolved in 1 mL of isopropanol. One drop of a 5 M solution of HCl in iPrOH is added. After addition of 10 mL of dry ether, a white precipitate appears which is filtered off, washed with dry ether, and dried in vacuo. Yield: 25.5 mg (0.60 mmoles; 85%; MW 427 as the free base).
¹H-NMR-Spectrum (MeOH-d4) FS121
3.09 (t, 5.8 Hz, 2H), 3.15 (br s, 1H), 3.51 (br s, 2H), 3.86 (br s, 1H), 4.08 (br s, 1H), 4.45 (br s, 1H), 7.25 (dd, 8.6 Hz, 1.9 Hz, 1H), 7.34-7.41 (m, 2H), 7.46 (td, 7.3 Hz, 1.6 Hz, 1H), 7.60 (dd, 8.2 Hz, 1.0 Hz, 1H)7.71 (dd, 7.7 Hz, 1.6 Hz, 1H), 7.75 (d, 1.7 Hz, 1H)
¹H-NMR-Spectra
2-(9-Bromo-6-oxo-6,7-dihydro-*5H*-benzo[2,3]azepino[4,5-b]indol-5-yl)-N-(2-methylamino-1-yl-ethyl)-acetamide (MeOH-d4)
2.68 (s, 3H), 3.10 (t, 5.6 Hz, 2H), 3.15 (br s, 1H), 3.50 (br s, 2H), 3.83 (br s, 1H), 4.16 (br s, 1H), 4.41 (br s, 1H), 7.32 (d, 8.6 Hz, 1H), 7.43 (dd, 7.5 Hz, 1.2 Hz, 1H), 7.42 (td, 8.2 Hz, 1.6 Hz, 1H), 7.56 (dd, 8.2 Hz, 1.0 Hz, 1H), 7.67 (dd, 7.7 Hz, 1.6 Hz, 1H), 7.72 (d, 1.6 Hz, 1H)

### FS116 = N-(3-Amino-1-yl-propyl)-2-(9-bromo-6-oxo-6,7-dihydro-5H-benzo[2,3]azepino[4,5-b]indol-5-yl)-acetamide (MeOH-d4)

1.81 (br t, 2H), 2.93 (br t, 2H), 3.15 (br s, 1H), 3.87 (br s, 1H), 4.15 (br s, 1H),4.38 (br s, 1H), 7.23 (dd, 8.6 Hz, 1.9 Hz, 1H), 7.33 (d, 8.6 Hz, 1H), 7.37 (dd, 7.5 Hz, 1.3 Hz, 1H), 7.43 (td, 8.2 Hz, 1.6 Hz, 1H), 7.58 (dd, 8.2 Hz, 1.1 Hz, 1H), 7.68 (dd, 7.7 Hz, 1.6 Hz, 1H), 7.72 (d, 1.7 Hz, 1H)

### FS 122 = N-(4-Amino-1-yl-butyl)-2-(9-bromo-6-oxo-6,7-dihydro-5H-benzo[2,3]azepino[4,5-b]indol-5-yl)-acetamide (MeOH-d4)

1.26-1.33 (m, 4H), 2.55 (t, 7.4 Hz, 2H), 2.87 (br s, 3H), 3.47 (br s, 1H), 3.79 (br s, 1H), 4.08 (br s, 1H), 6.89 (dd, 8.6 Hz, 1.9 Hz, 1H), 6.98-7.04 (m, 2H), 7.08 (td, 7.3 Hz, 1.6 Hz, 1H), 7.23 (dd, 8.2 Hz, 1.0 Hz, 1H), 7.34 (dd, 7.7 Hz, 1.6 Hz, 1H), 7.37 (d, 1H, 1.8 Hz)

### FS123 = N-(4-(3-Ammo-1-yl-propylammo)-1-yl-butyl)-2-(9-bromo-6-oxo-6,7-dihydro-5H-benzo[2,3]azepino[4,5-b]indol-5-yl)-acetamide (MeOH-d4)

1.60 (br s, 2H), 1.70-1.77 (m, 2H), 2.06-2.13 (m, 2H), 3.03-3.15 (m, 7H), 3.16-3.41 (m, 2H), 3.91 (br s, 1H), 4.19 (br s, 1H), 4.46 (br s, 1H), 7.30 (dd, 8.6 Hz, 1.8 Hz, 1H), 7.39-7.45 (m, 2H), 7.50 (td, 7.4 Hz, 1.5 Hz, 1H), 7.61 (dd, 8.1 Hz, 0.7 Hz, 1H), 7.75 (dd, 7.7 Hz, 1.5 Hz, 1H), 7.79 (d, 1.6 Hz, 1H)

### FS124 N-(3-(4-Amino-1-yl-butylamino)-1-yl-propyl)-2-(9-bromo-6-oxo-6,7-dihydro-5H-benzo[2,3]azepino[4,5-b]indol-5-yl)-acetamide (MeOH-d4)

1.63 (br s, 4H), 1.86 (br s, 2H), 2.70 (br s, 1H), 2.89 (br s, 2H), 2.97-2.99 (m, 2H), 3.08 (br s, 1H), 3.37-3.43 (m, 3H), 4.00 (br s, 1H), 4.19 (br s, 1H), 4.38 (br s, 1H), 7.32-7.39 (m, 3H), 7.44 (td, J= 8.3 Hz, 1.7 Hz, 1H), 7.55 (d, J=8.2 Hz, 1H), 7.67-7.71 (m, 1H), 7,73 (d, J= 1.8 Hz, 1H).

### FS150 = 2-(9-Cyano-6-oxo-6,7-dihydro-5H-benzo[2,3]azepino[4,5-b]indol-5-yl)-N-(2-methylamino-1 -yl-ethyl)-acetamide (MeOH-d4)

2.70 (s, 3H), 3.12 (t, 5.7 Hz, 2H), 3.21 (br s, 1 H), 3.51 (br s, 2H), 3.96 (br s, 1H), 4.23 (br s, 1H), 4.41 (br s, 1H), 7.38-7.44 (m, 2H), 7.48 (td, 8.3 Hz, 1.5 Hz, 1H), 7.55 (dd, 8.9 Hz, 0.4 Hz, 1H), 7.60 (dd, 8.2 Hz, 1.0 Hz, 1H), 7.72 (dd, 7.7 Hz, 1.6 Hz, 1H), 8.06 (d, 0.8 Hz, 1H)

### FS142 = N-(2-Methylamino-1-yl-ethyl)-2-(6-oxo-6,7-dihydro-5H-benzo[2,3]azepino[4,5-b]indol-5-yl)-acetamide (MeOH-d4)

2.69 (s, 3H), 3.11 (t, 5.6 Hz, 2H), 3.16 (br s, 1H), 3.52 (br s, 2 H), 3.88 (br s, 1H), 4.19 (br s, 1H), 4.42 (br s, 1H), 7.04-7.07 (m, 1H), 7.12-7.20 (m, 1H), 7.29-7.44 (m, 3H), 7.56-7.60 (m, 2H), 7.70 (dd, 7.5 Hz, 1.8 Hz, 1H)

### FS151 = N-[2-(9-Bromo-6-oxo-6,7-dihydro-5H-benzo[2,3]azepino[4,5-b] indol-5-yl)-acetyl]-piperazine (DMSO-d6)

3.08 (br s, 5H), 3.66 (br s, 4H), 3.92 (br s, 1H), 4.38 (br s, 1H), 4.70 (br s, 1H, 7.28 (d, 7.4 Hz, 1H), 7.30-7.45 (m, 4H), 7.72 (d, 6.2 Hz, 1H), 7.92 (s, 1H), 9.33 (br s, 2H), 12.01 (s, 1H).

### 2-(9-Bromo-6-oxo-6,7-dihydro-5H-benzo[2,3]azepino[4,5-b]indol-5-yl)-acetic acid hydrazide (DMSO-d6)

3.11 (br s, 1H), 3.96 (br s, 2H), 4.24 (d, J=3.8 Hz, 2H), 4.34 (br s, 1H), 7.28 (dd, J=8.6 Hz, 1.9 Hz, 1H), 7.37-7.43 (m, 2H), 7.47 (td, J= 8.3 Hz, 1.6 Hz, 1H), 7.58 (d, 7.7 Hz, 1H), 7.71 (dd, J= 7.7 Hz, 1.3 Hz, 1H), 7.94 (d, J= 1.7 Hz), 1H), 9.23 (br s, 1H), 11.92 (s, 1H)

### Screening of potential inhibitors against TryS

The compounds were screened against TryS at 10 µM compound concentration. The inhibitory effect of each compound on TryS was measured in triplicates. Obvious outliers from the triplicates were removed. If the standard deviation of the two remaining inhibition values exceeded 25 % the measurement was repeated.

The screen was performed under the following conditions:
90.75 nM TryS was incubated with 0.675 mM GSH, 6.75 mM spermidine, 45 µM ATP, and 10 µM compound in a total volume of 80 µl 100 mM HEPES pH 7.5, 10 mM MgSO₄, 0.1 mM EDTA, 5 mM DTT at room temperature for 60 min. Reaction was stopped by addition of 10 µl malachitgreen reagent. A650 was measured after 20 min. No compound was present in the positive controls (C+). Neither GSH nor spermidine was present in the negative controls (C-).

| Compound ID | inhibitory effect (%) |
|---|---|
| FS 100 | 97.88 |
| FS 115 | 10.88 |
| FS 116 | 85.13 |
| FS 121 | 96.84 |
| FS 122 | 54.26 |
| FS 123 | 47.63 |
| FS 124 | 47.58 |

### References

1. Fairlamb AH, and Cerami A (1985). Identification of a novel, thiol-containing co-factor essential for glutathione reductase enzyme activity in trypanosomatids. Mol. Biochem. Parasitol. 14: 187-198.
2. Flohé L (1998). The Achilles' heel of trypanosomatids: trypanothione-mediated hydroperoxide metabolism. Biofactors 8: 87-91. Review.
3. Nogoceke E, Gommel DU, Kiess M, Kalisz HM, Flohe L (1997). A unique cascade of oxidoreductases catalyses trypanothione-mediated peroxide metabolism in Crithidia fasciculata. Biol. Chem. 378: 827-836.
4. Krauth-Siegel RL, Meiering SK, and Schmidt RH (2003). The parasite-specific trypanothione metabolism of Trypanosome and Leishmania. Biol. Chem. 384: 539-549. Review.
**5.** Oza SL, Tetaud E, Ariyanayagam MR, Wamon SS, and Fairlamb AH (2002). A single enzyme catalyses formation of trypanothione from glutathione and spermidine in Trypanosoma cruzi. J. Biol. Chem. 277: 35853-35861.
**6.** Comini M, Menge U, Flohe L (2003). Biosynthesis of trypanothione in Trypanosoma brucei brucei. Biol. Chem. 384: 653-656.
**7.** Ariyanayagam MR, Oza SL, Guther ML, Fairlamb AH (2005). Phenotypic analysis of trypanothione synthetase knockdown in the African trypanosome. Biochem. J. Jul 12; [Epub ahead of print]
**8.** Pineyro MD, Pizarro JC, Lema F, Pritsch O, Cayota A, Bentley GA, Robello C (2005). Crystal structure of the tryparedoxin peroxidase from the human parasite Trypanosoma cruzi. J. Struct. Biol. 150: 11-22.
**9.** Dormeyer M, Reckenfelderbäumer N, Lüdemann H, and Krauth-Siegel RL (2001). Trypanothione-dependent synthesis of deoxyribonucleotides by Trypanosoma brucei ribonucleotide reductase. J. Biol. Chem. 276: 10602-10606.
**10.** Krieger S, Schwarz W, Ariyanayagam MR, Fairlamb AH, Krauth-Siegel RL, and Clayton C (2000). Mol. Microbiol. 35: 542-552.
**11.** Tovar J, Cunningham ML, Smith AC, Croft SL, and Fairlamb AH (1998). Down-regulation of Leishmania donovani trypanothione reductase by heterologous expression of a trans-dominant mutant homologue: effect on parasite intra-cellular survival. Proc. Natl. Acad. Sci. USA 95: 5311-5316.
**12.** Wilkinson SR, Horn D, Radhika Prathalingam S, and Kelly JM (2003). RNA interference identifies two hydroperoxide metabolizing enzymes that are essential to the bloodstream form of the African trypanosome. J. Biol. Chem. 278: 31640-31646.
**13.** Comini MA, Guerrero SA, Haile S, Menge U, Lünsdorf H, Flohé L (2004). Validation of Trypanosoma brucei trypanothione synthetase as drug target. Free Radic. Biol. Med. 36: 1289-1302.
14. Castro H, Sousa C, Novais M, Santos M, Budde H, Cordeiro-da-Silva A, Flohé L, Tomás AM (2004). Two linked genes of Leishmania infantum encode tryparedoxins localized to cytosol and mitochondrion. Mol. Biochem. Parasitol. 136: 137-147.
15. Irsch T, Krauth-Siegel RL (2004). Glyoxalase II of African trypanosomes is trypanothione-dependent.J. Biol. Chem. 279: 22209-22217.
16. Comini M, Menge U, Wissing J, and Flohé L (2005). Trypanothione synthesis in Crithidia revisited. J. Biol. Chem. 280: 6850-6860.

## Claims

1. Compounds having the general formula (1): 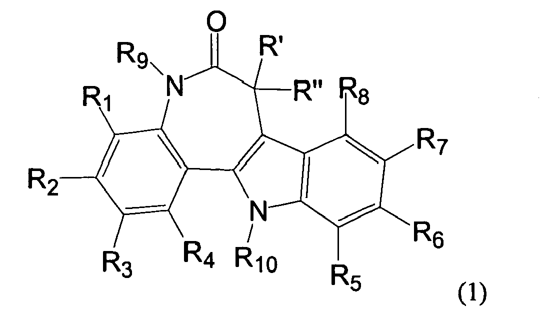 wherein
R¹-R⁸ R⁸¹, R⁸² represent independently of each other -R¹¹ ,-R¹² ,-R¹³, -R¹⁴, -R¹⁵ -R¹⁶,-R¹⁷, -R¹⁸ , -CH2-R¹⁹, -CH₂-R²⁰ , -CH₂-R²¹, -CH₂-R²² , -CHR²³ R²⁴, -CHR²⁵R²⁶,-CHR²⁷R²⁸, -CHR⁹ R³⁰ -CR³¹ R³² R³³ -CR³⁴ R³⁵ R³⁶, -CH₂-CH₂-R³⁷, -CH₂-CH₂-R³⁸,-CH₂-CH-R³⁹ R⁴⁰, -CH₂-CH-R⁴¹R⁴²_{'} -CR⁴³R⁴⁴-CR⁴⁵R⁴⁶R⁴⁷, -CR⁴⁸R⁴⁹-CR⁵⁰R⁵¹ R⁵²,-(CH₂)ₛ-R⁶⁵, -(CH₂)ₜ-R⁶⁶, -CR⁵³R⁵⁴-CR⁵⁵R⁵⁶-CR⁵⁷R⁵⁸R⁵⁹, -CR⁶⁰R⁶¹-CR⁶²R⁶³-CR⁶⁴R⁶⁵R⁶⁶, -(CH₂)_{q}-C R⁶⁰R⁶¹- (CH₂)ᵣR⁶⁴, or -H;
R⁹ represents -CHR⁶⁸-CHR⁶⁷-CO-NR⁸¹R⁸², -CHR⁶⁷-CO-NR⁸¹R⁸², -CO-NR⁸¹R⁸² , -CO-NR⁸¹R⁸², -CHR⁶⁸-CHR⁶⁷-CO-R⁸¹, -CHR⁶⁷-CO-R⁸¹, -CO-R⁸¹, -CHR⁶⁸-CHR⁶⁷-CO-OR⁸¹, -CHR⁶⁷-CO-OR⁸¹, or -CO-OR⁸¹;
R', R" and R¹⁰ represent independently of each other -H, -CPh₃, -CH₃, -C₂H₅, -C₃H₇,-cyclo-C₃H₇, -CH(CH₃)₂, -C₄H₉, -CH₂-CH(CH₃)₂, -CH(CH₃)-C₂H₅, -C(CH₃)₃, -C₅H₁₁,-C₆H₁₃, -C₇H₁₅, -C₈H₁₇, -C₉H₁₉, -C₁₀H₂₁, -Ph, -CH₂-Ph, -CH=CH₂, -CH₂-CH=CH₂, -C(CH₃)=CH₂, -CH=CH-CH₃, -C₂H₄-CH=CH₂, -CH=C(CH₃)₂, -C≡CH, -C≡C-CH₃, or -CH₂-C≡CH;
R¹¹- R⁸⁰ represent independently of each other-H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃,-SC₂H₅, -SC₃H₇, -S-cyclo-C₃H₅, -SCH(CH₃)₂, -NO₂, -F, -Cl, -Br, -I, -N₃, -CN, -OCN,-NCO, -SCN, -NCS, -CHO, -COCH₃, -COC₂H₅, -COC₃H₇, -CO-cyclo-C₃H₅,-COCH(CH₃)₂, -COC(CH₃)₃, -COOH, -COCN, -COOCH₃, -COOC₂H₅, -COOC₃H₇,-COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂,-CON(C₂H₅)₂, -CON(C₃H₇)₂, -CON(cyclo-C₃H₅)₂, -CON[CH(CH₃)₂]₂, -CON[C(CH₃)₃]₂,-NH₂ , -NHCH₃, -NHC₂H₅, -NHC₃H₇, -NH-cyclo-C₃H₅, -NHCH(CH₃)₂, -NHC(CH₃)₃,-N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅,-SO₂C₃H₇, -SO₂-cyclo-C₃H₅, -SO₂CH(CH₃)₂, -SO₂C(CH₃)₃, -SO₃CH₃, -SO₃C₂H₅,-SO₃C₃H₇, -SO₃-cyclo-C₃H₅, -SO₃CH(CH₃)₂, -SO₃C(CH₃)₃, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇,-NH-CO-NH-cyclo-C₃H₅, -NH-CO-NH[CH(CH₃)₂], NH-CO-NH[C(CH₃)₃], -NH-CON(CH₃)₂, -NH-CO-N(C₂H₅)₂, -NH-CO-N(C₃H₇)₂, -NH-CO-N(cyclo-C₃H₅)₂, -NH-CON[CH(CH₃)₂]₂, -NH-CO-N[C(CH₃)₃]₂, -NH-CS-NH₂, -NH-CS-NHCH₃, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)₂, -NH-CS-N(C₃H₇)₂, -NH-CS-N(cyclo-C₃H₅)₂, -NH-CS-N[CH(CH₃)₂]₂, -NH-CS-N[C(CH₃)₃]₂, -NH-C(=NH)-NH₂, -O-CO-NH₂, -O-CONHCH₃, -O-CONHC₂H₅, -O-CONHC₃H₇, -O-CONH-cyclo-C₃H₅, -O-CONH[CH(CH₃)₂], -O-CONH[C(CH₃)₃], -O-CON(CH₃)₂, -O-CON(C₂H₅)₂, -O-CON(C₃H₇)₂, -O-CON(cyclo-C₃H₅)₂, -O-CON[CH(CH₃)₂]₂, -O-CON[C(CH₃)₃]₂, -CH₂F,-CF₂H, -CF₃, -CH₂Cl, -CHCl₂, -CCl₃, -CH₂Br, -CHBr₂, -CBr₃, -CPh₃, -CH₂-CH₂F, -CH₂-CHF₂, -CH₂-CF₃, -CH₂-CH₂Cl, -CH₂-CHCl₂, -CH₂-CCl₃, -CH₂-CH₂Br, -CH₂-CHBr₂,-CH₂-CBr₃, -CH₃, -C₂H₅, -C₃H₇, -cyclo-C₃H₅, -CH(CH₃)₂, -C(CH₃)₃, -C₄H₉, -CH₂-CH(CH₃)₂, -CH(CH₃) -C₂H₅, -C₅H₁₁, -C₆H₁₃, -C₇H₁₅, -C₈H₁₇, -C₉H₁₉, -C₁₀H₂₁, -Ph, -CH₂-Ph, -CH=CH₂, -CH₂-CH=CH₂, -C(CH₃)=CH₂, -CH=CH-CH₃, -C₂H₄-CH=CH₂,-CH=C(CH₃)₂, -C≡CH, -C≡C-CH₃, -CH₂-C≡CH; -CH₂-CH₂S-CH₃, -CH₂-OH, -CH(OH)CH₃, -CH₂-NH₂, -CH₂-CO-NH₂, -CH₂-CH₂-CO-NH₂, -CH₂-SH, - (CH₂)₃-NH-C(=NH)NH₂, -(CH₂)₄-NH₂, -CH₂-COOH, or -CH₂-CH₂-COOH;
R⁶⁷ and R⁶⁸ represent independently of each other -R⁶⁹, -R⁷⁰, -CH₂-R⁶⁹, -CHR⁷⁰R⁷¹,-CR⁷²R⁷³R⁷⁴, -(CH₂)ₙ-R⁷⁵, -(CH₂)ₚ-CHR⁷⁶ R⁷⁷, -(CH₂)ₘ-CR⁷⁸ R⁷⁹R⁸⁰, -C₆H₄-R⁷², or -CH₂-C₆H₄-R⁷³,
R⁶⁹ - R⁸⁰ also represent independently of each other 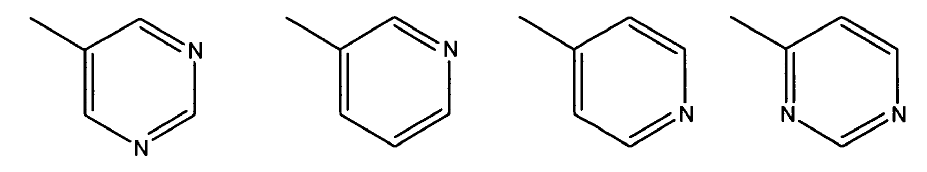 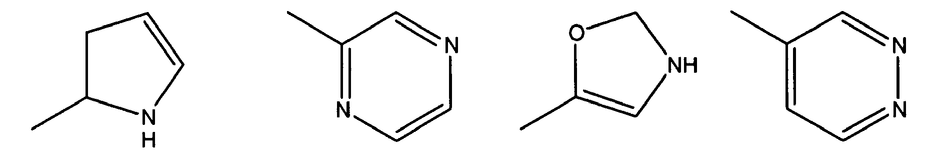 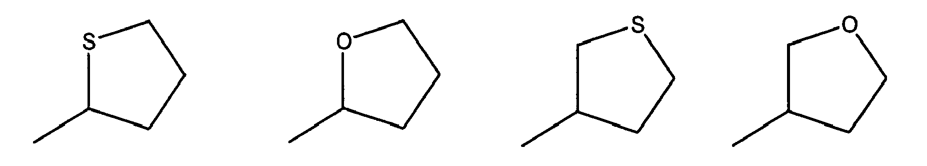 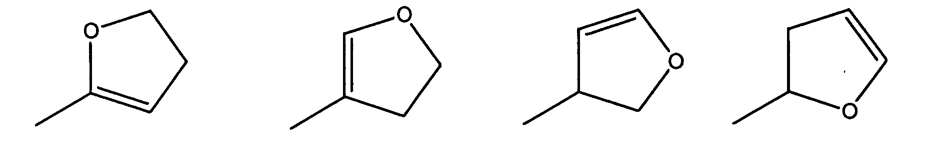 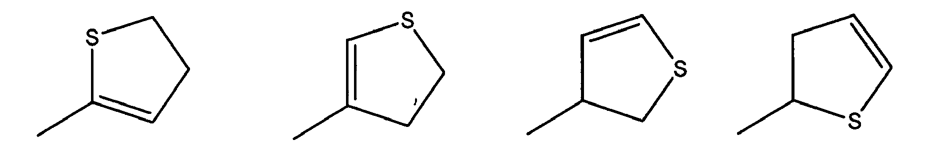 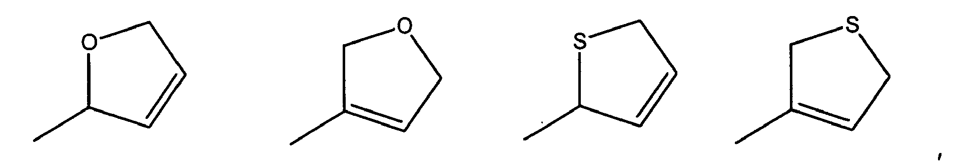 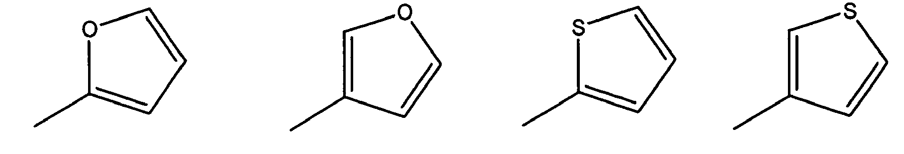 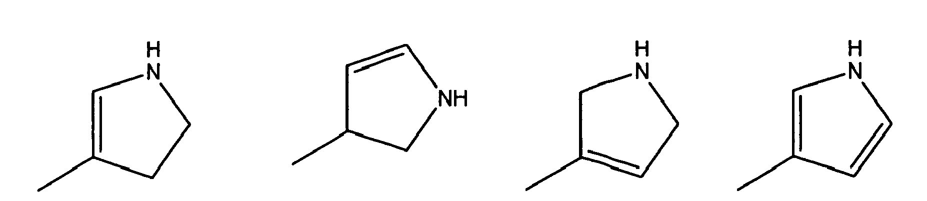 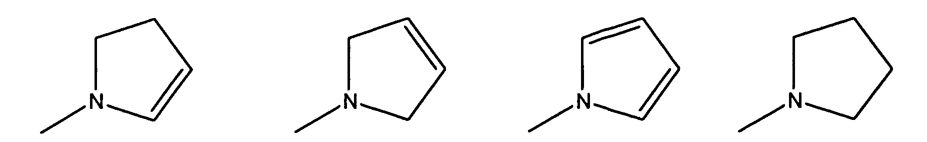 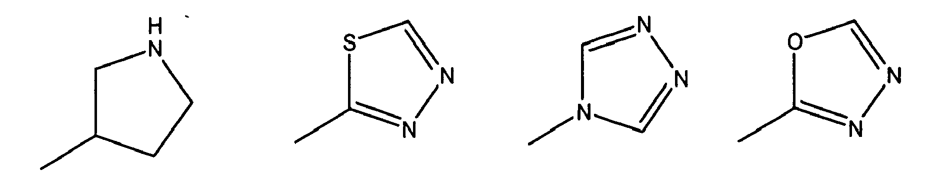 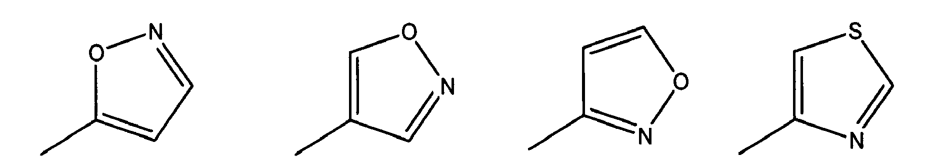 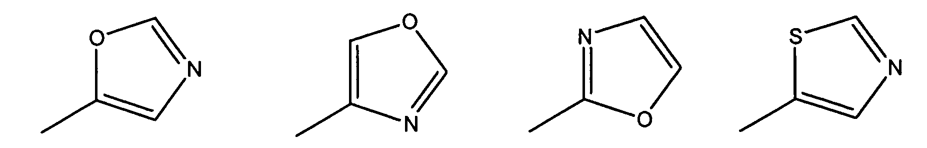 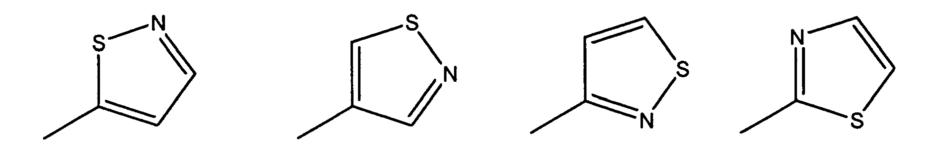 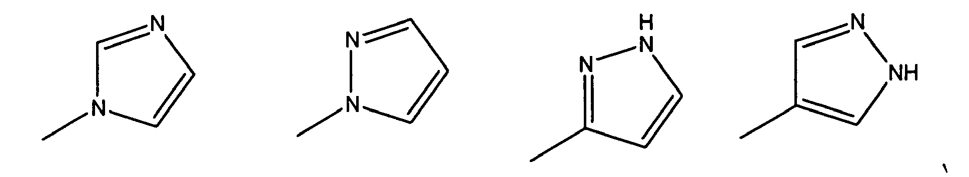 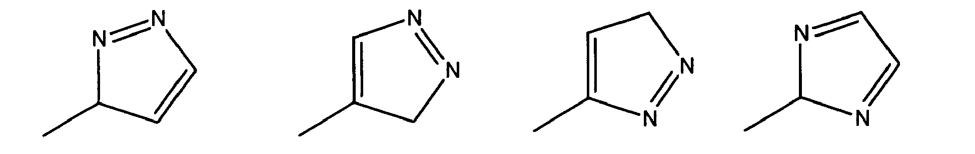 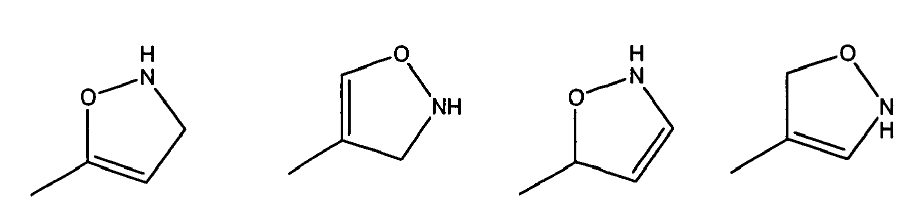 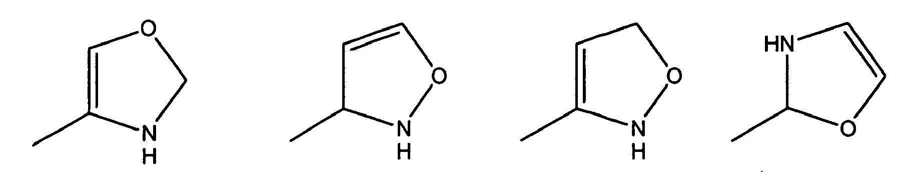 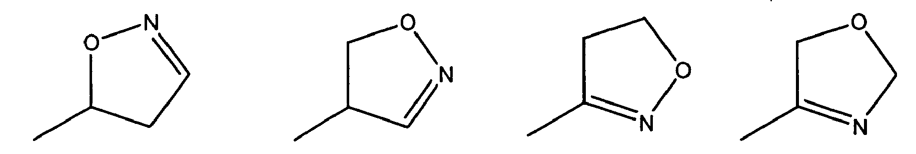 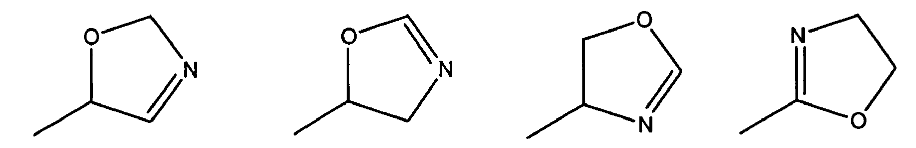 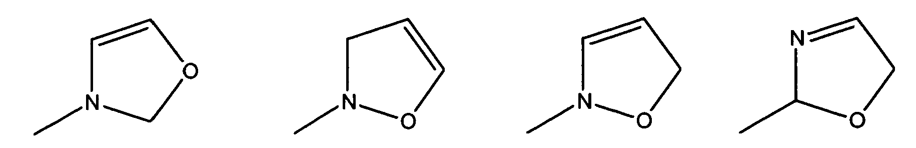 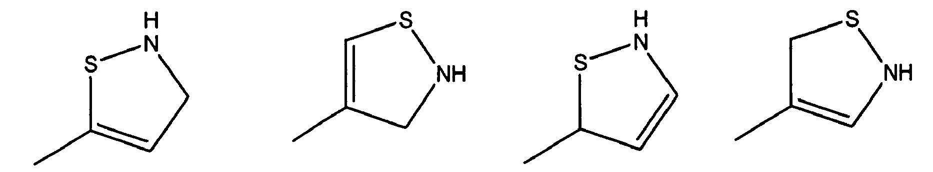 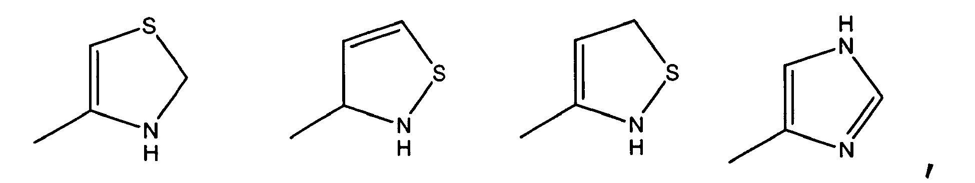 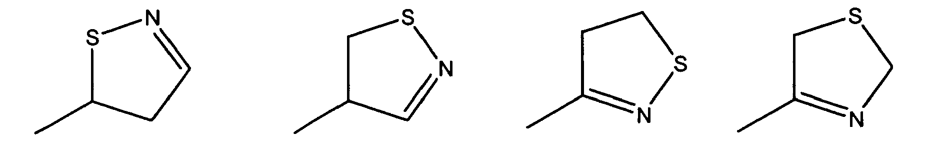 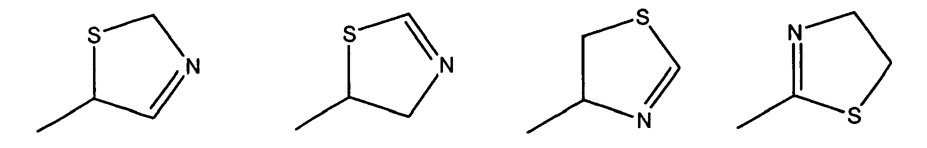 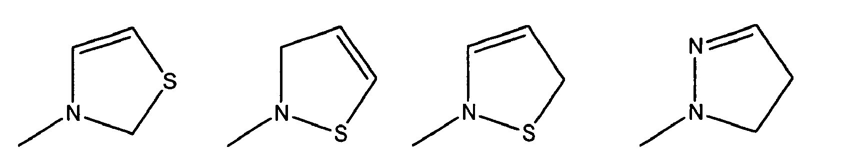 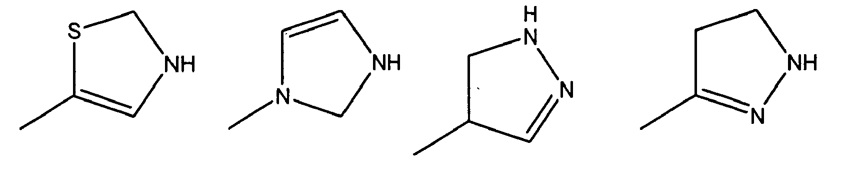 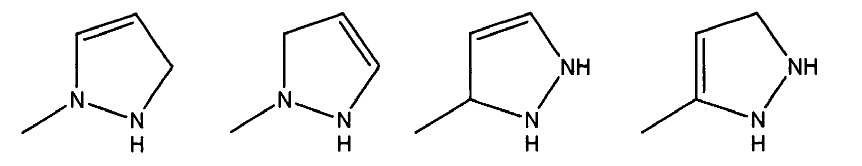 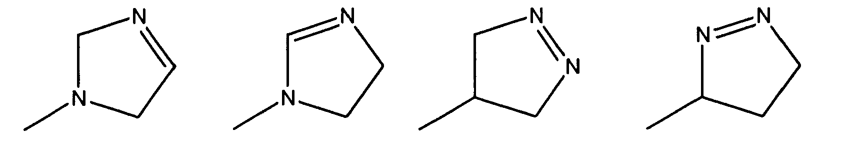 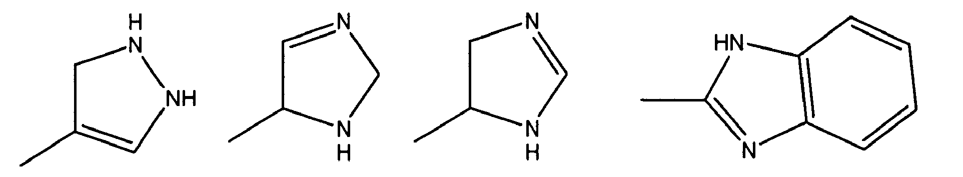 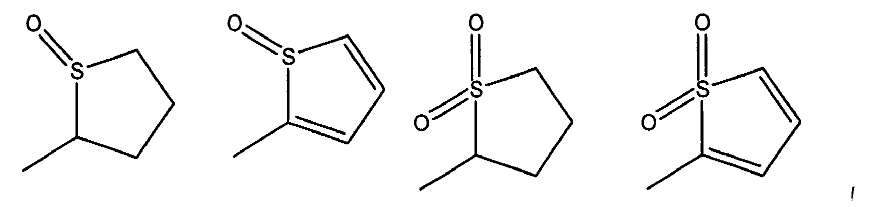 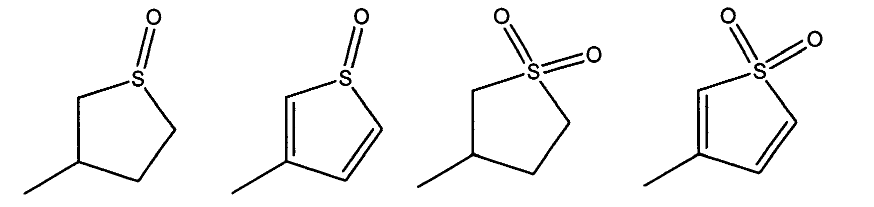 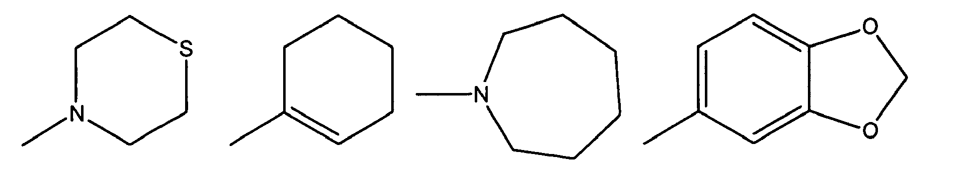 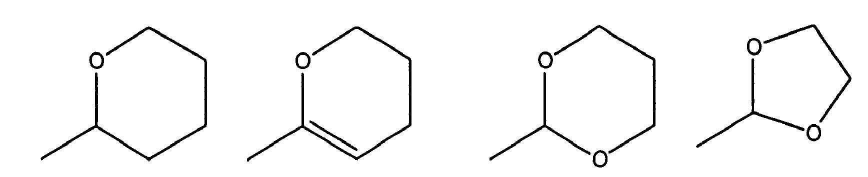 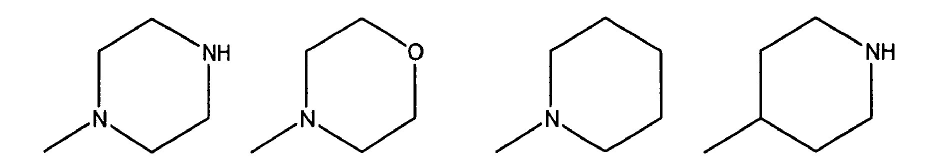 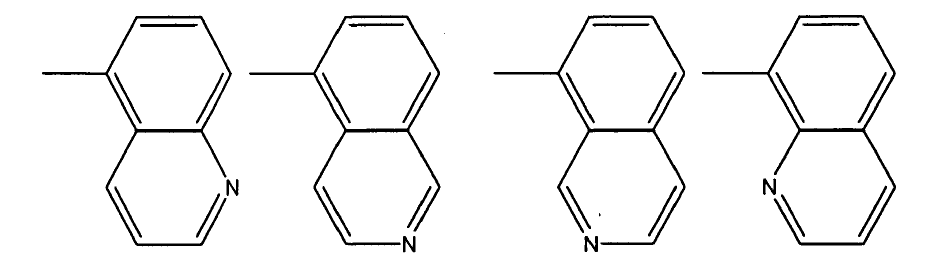 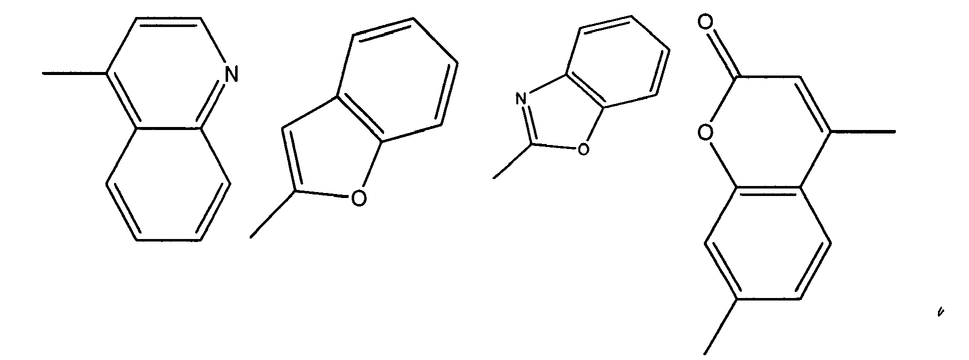 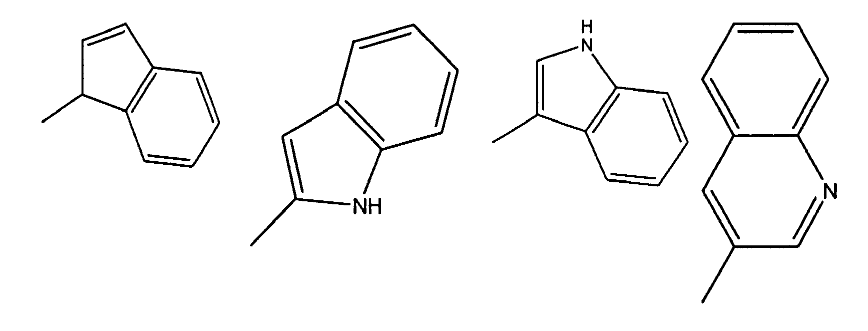 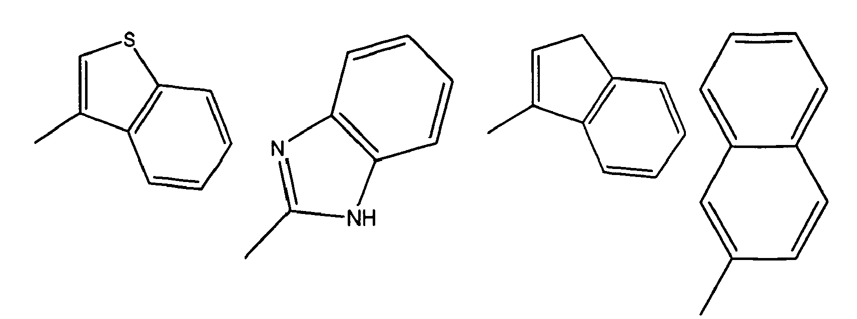 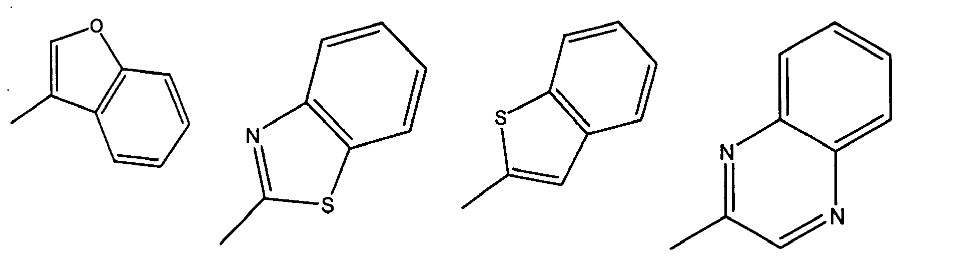 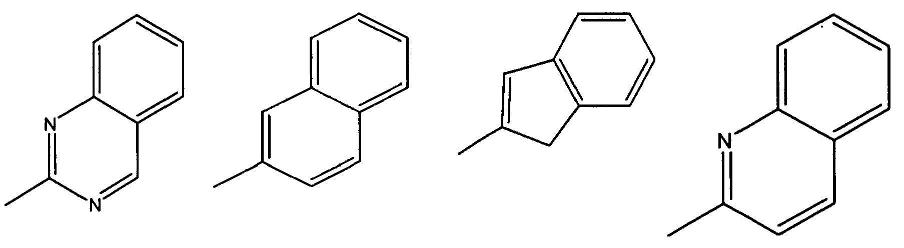 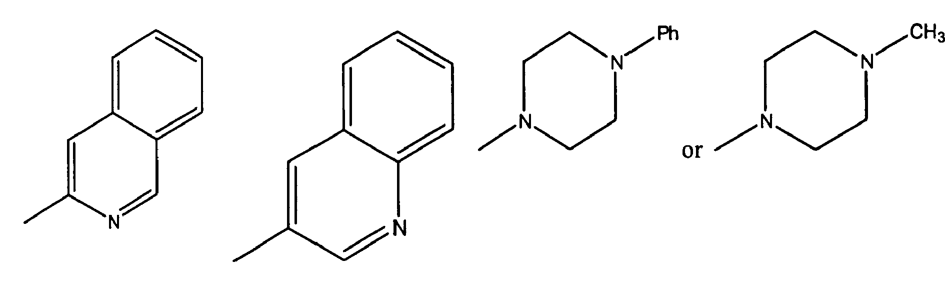
NR⁸¹R⁸² may also represent 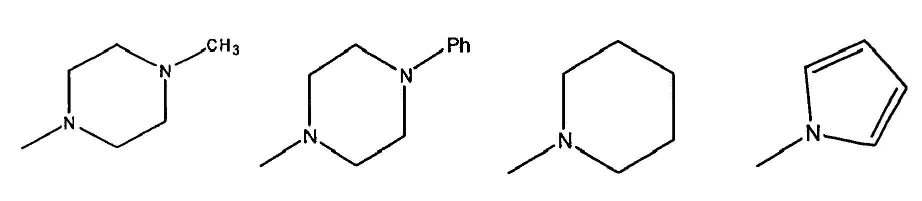 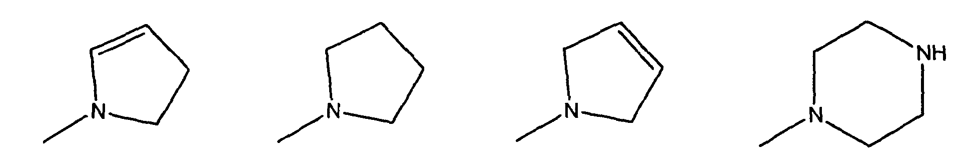 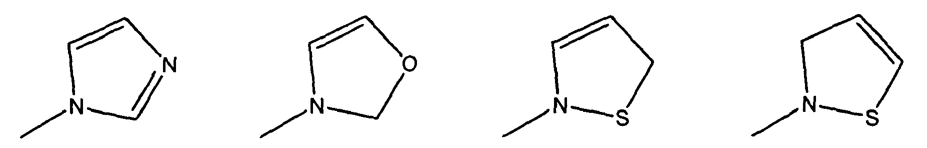 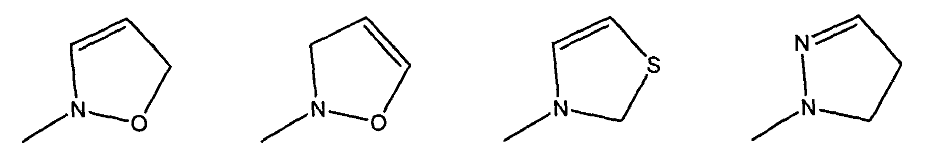 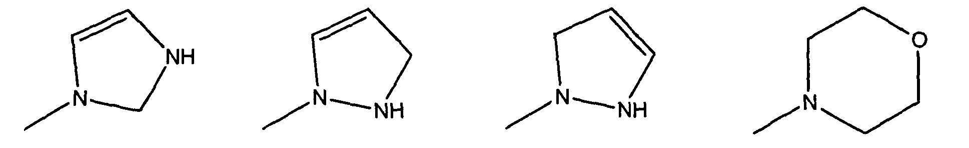 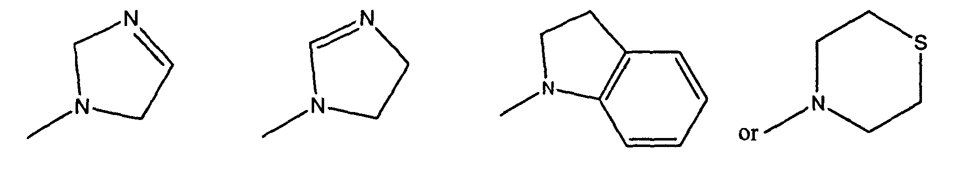
m, n, p, q, r, s, t are independently of each other integer from 0-10; and stereoisomeric forms, prodrugs, solvates, hydrates and/or pharmaceutically acceptable salts thereof.

2. Compounds according to claim 1 having the general formula (2), 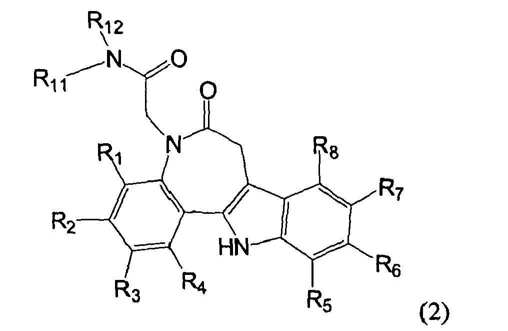 wherein R¹ - R⁸ and R¹¹ have the meanings as defined in claim 1.

3. Compounds according to claim 2 having the general formula (2), 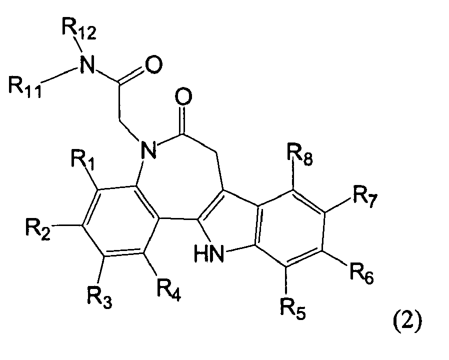 wherein, R¹ - R⁸ represent independently of each other -H, -CH₃, C₂H₅, -(CH₂)₂CH₃, -OCH₃, -OC₂H₅, -F, -Cl, -Br, -J, -SCH₃, -SC₂H₅, -S(CH₂)₂CH₃, -CN, -NO₂ -CO₂CH₃, -CO₂C₂H₅,-CO₂(CH₂)₂CH₃, -NH₂, -NHCH₃, -N(CH₃)₂, -NHC₂H₅, -N(C₂H₅)₂, -CHO, -COCH₃, -COC₂H₅, -CO(CH₂)₂CH₃, -S(O)CH₃, -SO₂CH₃, -SO₂NH₂, -SO₂NHCH₃, -SO₂N(CH₃)₂ -SO₂NHC₂H₅,-or SO₂N(C₂H₅)₂,
and R¹¹ and R¹² represent independently of each other -H, -CH₃, -C₂H₅, -(CH₂)₂CH₃, -OCH₃,-OC₂H₅, -COCH₃, -COC₂H₅, -CO(CH₂)₂CH₃, -NH₂, -NHCH₃, -N(CH₃)₂, -NHC₂H₅, -N(C₂H₅)₂, -CHO, -COCH₃, -COC₂H₅, -CO(CH₂)₂CH₃, -SO₂CH₃, -SO₂C₂H₅, -SO₂ (CH₂)₂CH₃, - SO₂Ph,-C(NH)NH₂, -(CH₂)₂NH₂, -(CH₂)₂OH, -(CH₂)₂SH, -(CH₂)₂NHCH₃, -(CH₂)₂NHC₂H₅,-(CH₂)₂NH-(CH₂)₂CH₃, -(CH₂)₂NH-(CH₂)₂-NH₂, -(CH₂)₂NH-(CH₂)₃-NH₂, -(CH₂)₂NH-(CH₂)₄-NH₂, -(CH₂)₃NH-(CH₂)₂-NH₂, -(CH₂)₃NH-(CH₂)₃-NH₂, -(CH₂)₃NH-(CH₂)₄-NH₂,-(CH₂)₂NHCOCH₃, -(CH₂)₂NHCHO, -(CH₂)₂NHCOC₂H₅, -(CH₂)₂NHSO₂CH₃,-(CH₂)₂NHSO₂Ph, -(CH₂)₂NHCOPh, -(CH₂)₂NHCO₂CH₃, -(CH₂)₂NHCO₂C₂H₅, or -(CH₂)₂NHCO₂Ph,
and -NR¹¹R¹² preferably also represents piperazine.

4. Compounds according to claim 1, 2, or 3 wherein the compound is selected from the group comprising:
2-(9-Bromo-6-oxo-6,7-dihydro-5*H*-benzo[2,3]azepino[4,5-b]indol-5-yl)-N-(2-methylamino-1-yl-ethyl)-acetamide
N-(2-Amino-1-yl-ethyl)- 2-(9-bromo-6-oxo-6,7-dihydro-5*H*-benzo[2,3]azepino[4,5-b]indol-5-yl)-acetamide
N-(3-Amino-1-yl-propyl)-2-(9-bromo-6-oxo-6,7-dihydro-5*H*-benzo[2,3]azepino[4,5-b]indol-5-yl)-acetamide
N-(4-Amino-1-yl-butyl)-2-(9-bromo-6-oxo-6,7-dihydro-5*H*-benzo[2,3]azepino[4,5-b]indol-5-yl)-acetamide
N-(4-(3-Amino-1-yl-propylamino)-1-yl-butyl)-2-(9-bromo-6-oxo-6,7-dihydro-5*H-*benzo[2,3]azepino[4,5-b]indo1-5-yl)-acetamide
N-(3-(4-Amino-1-yl-butylamino)-1-yl-propyl)-2-(9-bromo-6-oxo-6,7-dihydro-*5H-*benzo[2,3]azepino[4,5-b]indol-5-yl)-acetamide
2-(9-Cyano-6-oxo-6,7-dihydro-*5H*-benzo[2,3]azepino[4,5-b]indol-5-yl)-N-(2-methylamino-1-yl-ethyl)-acetamide
N-(2-Methylamino-1-yl-ethyl)-2-(6-oxo-6,7-dihydro-*5H-*benzo[2,3]azepino[4,5-b]indol-5-yl)-acetamide
2-(9-Methyl-6-oxo-6,7-dihydro-*5H*-benzo[2,3]azepino[4,5-b]indol-5-yl)-N-(2-methylamino-1-yl-ethyl)-acetamide
N-[2-(9-Bromo-6-oxo-6,7-dihydro-*5H*-benzo[2,3]azepino[4,5-b]indol-5-yl)-acetyl]-piperazine
2-(9-Bromo-6-oxo-6,7-dihydro-*5H*-benzo[2,3]azepino[4,5-b]indol-5-yl)-acetic acid hydrazide

5. Compounds according to any previous claim for use as a pharmaceutically active agent.

6. Use of a compound according to any one of claims 1 to 4 as inhibitor of a trypanothione-dependent reaction and especially a reaction comprising trypanothione synthetase or trypanothione reductase.

7. Use of a compound according to claim 6 for prophylaxis and/or treatment of diseases caused by parasites that depend on trypanothione.

8. Use of a compound according to any one of the claims 1 to 4 for prophylaxis and/or treatment of South American trypanosomiasis, African trypanosomiasis, sleeping sickness, Kala-Azar, visceral leishmaniasis, Baghdad boil or Aleppo boil, cutaneous leishmaniasis (CL), espundia, Chagas disease, mucocutaneous leishmaniasis (MCL), trichomoniasis, urogenital trichomonosis, giardiasis, lamblia dysentery, amoebiasis, primary amebic meningoencephalitis (PAM), keratitis or meningitis, coccidiosis, sarcosporidosis, toxoplasmosis, Malaria tropica, Malaria tertiana, Malaria quartana, pneumocystis carinii, pneumonia, pneumocystosis, Balantidium dysentery, and/or oriental sore.

9. Use of a compound according to any one of the claims 1 to 4 in combination with an antitrypanosomal drug especially Melarsoprol, Nifurtimox, Suramine, Atoxyl, Tryparsamide, DFMO, Megazole, and/or Pentamidine.

10. Pharmaceutical compositions comprising at least one compound according to any one of the claims 1 to 4 as an active ingredient, together with at least one pharmaceutically acceptable carrier, excipient and/or diluent.
